# EUROPÄISCHE PATENTANMELDUNG

(11) **EP 2 163 613 A2**
(43) Veröffentlichungstag der Anmeldung: **17.03.2010**
(21) Anmeldenummer: 09167910.0
(22) Anmeldetag: 14.08.2009
(51) Int. Cl.: C12N 1/20

(54) **Verfahren zur Herstellung von organisch-chemischen Verbindungen unter Verwendung von verbesserten Stämmen der Familie Enterobacteriaceae**

(30) Priorität: 28.08.2008 DE 102008044768
(71) Anmelder: Evonik Degussa GmbH, 45128 Essen (DE)
(72) Erfinder: Rieping, Mechthild. Dr., 33619 Bielefeld (DE); Jerrentrup, Silke, 33607 Bielefeld (DE)

(57) **Zusammenfassung**

Die Erfindung betrifft ein Verfahren zur Herstellung von organisch-chemischen Verbindungen durch Fermentation von rekombinanten Mikroorganismen der Familie Enterobacteriaceae, **dadurch gekennzeichnet, dass** man
a) die die gewünschte organisch-chemische Verbindung produzierenden Mikroorganismen, in denen man das mdtO-Gen, für das Genprodukt kodierende Nukleotidsequenzen oder Allele verstärkt, insbesondere überexprimiert, in einem Medium kultiviert unter Bedingungen, bei denen die gewünschte organisch-chemische Verbindung im Medium oder in den Zellen angereichert wird, und
b) die gewünschte organisch-chemische Verbindung isoliert, wobei gegebenenfalls weitere Bestandteile der Fermentationsbrühe und/oder die Biomasse in ihrer Gesamtheit oder Anteilen (≥ 0 bis 100 %) im isolierten Produkt verbleiben oder vollständig entfernt werden.

## Beschreibung

Diese Erfindung betrifft ein Verfahren zur fermentativen Herstellung von organisch-chemischen Verbindungen unter Verwendung von rekombinanten Mikroorganismen der Familie Enterobacteriaceae, in denen das mdtO-Gen, welches für die Translokase-Komponente eines Multidrug-Transporters (MDT) kodiert, verstärkt, insbesondere überexprimiert wird, und diese Mikroorganismen.

### Stand der Technik

Organisch-chemische Verbindungen, mit denen insbesondere L-Aminosäuren, Ketosäuren, Vitamine, Nukleoside und Nukleotide und D-Aminosäuren gemeint sind, finden in der Humanmedizin, in der pharmazeutischen Industrie, in der Kosmetik, in der Lebensmittelindustrie und in der Tierernährung Anwendung.

Es ist bekannt, dass zahlreiche dieser Verbindungen durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht organisch-chemische Verbindungen überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren. Resistenz gegen das Tryptophan-Analogon 5-Methyl-DL-Tryptophan (5-MT) zeichnet zum Beispiel einen Stamm aus, der L-Tryptophan produziert.

Seit einigen Jahren werden ebenfalls Methoden der rekombinanten DNA-Technik zur gezielten Stammverbesserung organisch-chemische Verbindungen produzierender Stämme der Familie Enterobacteriaceae eingesetzt, indem man zum Beispiel einzelne Aminosäure-Biosynthesegene amplifiziert oder die Eigenschaften spezieller Gene verändert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)).

Neben Effluxpumpen mit hoher Substratspezifität transportieren sogenannte Multidrug-Transporter (MDT) oder multidrug efflux (mde) Transporter ein breites Spektrum strukturell sehr unterschiedlicher Substanzen, diese Transporter sind an der Vermittlung der multidrug resistance (MDR) beteiligt.

Die meisten mde-Transporter der gramnegativen Bakterien gehören der RND-Familie (resistance nodulation cell devision) an.

Effluxpumpen dieser Familie bestehen aus drei Teilen (Lomovskaya et al., Nature Biotechnology 20(12):1210-2 (2002)), dem in der Cytoplasmamembran lokalisierten Transporter, einem Porin in der äußeren Membran und einem Membranfusionsprotein, das die Verbindung zwischen Pumpe und Porin über den periplasmatischen Raum herstellt.

Von den drei Genen des Operons mdtNOP kodiert das mdtO-Gen für die Translokase-Komponente des Multidrug-Transporters und Sequenz-Analysen identifizieren das Genprodukt MdtP des mdtP-Gens als ein Mitglied der outer membrane factor (OMF) Familie. Die Deletion des mdtP-Gens im E. coli Stamm W3110 führt zu einer erhöhten Empfindlichkeit gegen die Gifte Akriflavin, Puromycin und Tetraphenylarsoniumchlorid (Sulavik et al., Antimicrobial Agents and Chemotherapy 45(4):1126-36 (2001)). Das Genprodukt MdtN zeigt Homologien zur Familie der membrane fusion proteins (MFP) (Dinh et al., Journal of Bacteriology 176(13):3825-31 (1994)).

Die Nukleotidsequenz der Wildform des für die Translokase-Komponente eines Multidrug-Transporters (MDT) von Escherichia coli kodierenden mdtO-Gens ist in der Datenbank des National Center for Biotechnology Information (NCBI) der National Library of Medicin (Bethesda, MD, USA) unter der Zugangsnummer (accession number) NC_000913 (Region: 4299050 - 4301101 (komplementär)) allgemein verfügbar.

### Aufgabe der Erfindung

Die Erfinder haben sich die Aufgabe gestellt, neue Maßnahmen zur Herstellung erhöhter Mengen von organisch-chemischen Verbindungen, insbesondere L-Aminosäuren, bereitzustellen.

### Beschreibung der Erfindung

Gegenstand der Erfindung sind rekombinante organisch-chemische Verbindungen produzierende Mikroorganismen der Familie Enterobacteriaceae, die ein verstärktes oder überexprimiertes mdtO-Gen, das für die Translokase-Komponente eines Multidrug-Transporters (MDT) kodiert, oder für dessen Genprodukt kodierende Nukleotidsequenzen enthalten, und die vermehrt organisch-chemischen Verbindungen, insbesondere L-Aminosäuren, produzieren und in der Zelle oder bevorzugt ausscheiden und im Medium anreichern.

Als Ausgangspunkt für den Vergleich dienen jeweils die für das mdtO-Gen nicht rekombinanten Mikroorganismen, die kein verstärktes mdtO-Gen enthalten und an denen die erfindungsgemäße Verstärkung oder Überexpression nicht durchgeführt wurde.

Zu den erfindungsgemäßen Mikroorganismen (oder Ausgangs-Mikroorganismen) gehören insbesondere Mikroorganismen der Familie Enterobacteriaceae, in denen ein Polynukleotid verstärkt wird, das für ein Polypeptid kodiert, dessen Aminosäuresequenz zu mindestens 80% oder zu mindestens 90%, insbesondere zu mindestens 95%, bevorzugt zu mindestens 98% oder mindestens 99%, besonders bevorzugt zu 99,6% und ganz besonders bevorzugt zu 100% identisch ist mit einer Aminosäuresequenz, ausgewählt aus der Gruppe SEQ ID No. 2, SEQ ID No. 4 und SEQ ID No. 6, bevorzugt SEQ ID No. 2, wobei das Polypeptid die Aktivität der Translokase-Komponente eines Multidrug-Transporters (MDT) besitzt.

Die erfindungsgemäßen Mikroorganismen enthalten bevorzugt dem mdtO-Gen entsprechende isolierte Polynukleotide, ausgewählt aus der Gruppe:
a) Polynukleotid mit einer Nukleotidsequenz, ausgewählt aus SEQ ID No. 1, SEQ ID No. 3, SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1, SEQ ID No. 3 oder SEQ ID No. 5 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält,
wobei die Polynukleotide für die Translokase-Komponente eines Multidrug-Transporters (MDT) kodieren.

Gegenstand der Erfindung ist ebenso ein Verfahren zur fermentativen Herstellung und Überproduktion von organisch-chemischen Verbindungen unter Verwendung von rekombinanten Mikroorganismen der Familie Enterobacteriaceae, die insbesondere bereits vor der erfindungsgemäßen Verstärkung oder Überexpression organisch-chemische Verbindungen produzieren, und in denen mindestens das mdtO-Gen oder für dessen Genprodukt kodierende Nukleotidsequenzen verstärkt wird bzw. werden.

Die rekombinanten Mikroorganismen scheiden die gewünschten Verbindungen bevorzugt aus.

Zu den erfindungsgemäß eingesetzten Mikroorganismen zählen dabei nicht Wildtypstämme und häufig benutzte Laborstämme wie z. B. DH5α, DH5αmcr, W3110, JM105, MG1655, MC4100, Y1089, H560 und MM152, in denen das mdtO-Gen verstärkt oder Überexprimiert wird.

Diese produzierenden organische Verbindungen nur unter strikter Regulation entsprechend ihrem Wildtypstatus.

Mit dem Begriff "organisch-chemische Verbindung" sind organische Säuren, Vitamine, Nukleoside und Nukleotide und Aminosäuren und deren Salze gemeint. Weiterhin gehören dazu von Pyruvat abgeleitete Metabolite. Bei den organischen Säuren werden Ketosäuren, insbesondere Brenztraubensäure (Pyruvat), Bernsteinsäure (Succinat), Ketoisovaleriansäure und Ketoisocapronsäure, bevorzugt. Bei den Aminosäuren werden L-Aminosäuren, insbesondere die proteinogenen L-Aminosäuren und L-Homoserin, bevorzugt.

Unter "proteinogenen Aminosäuren" versteht man die Aminosäuren die in natürlichen Proteinen, das heißt in Proteinen von Mikroorganismen, Pflanzen, Tieren und Menschen vorkommen. Sie dienen als Struktureinheiten für Proteine, in denen sie über Peptidbindungen miteinander verknüpft sind.

Die Begriffe Protein und Polypeptid sind gegenseitig austauschbar.

Werden im folgenden proteinogene L-Aminosäuren erwähnt, sind damit eine oder mehrere der Aminosäuren einschließlich ihrer Salze, ausgewählt aus der Gruppe L-Asparaginsäure, L-Asparagin, L-Threonin, L-Serin, L-Glutaminsäure, L-Glutamin, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Arginin und L-Prolin gemeint. Besonders bevorzugt sind die L-Aminosäuren L-Threonin, L-Tryptophan, L-Lysin, L-Valin, L-Isoleucin und L-Homoserin.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Verstärkung oder Überexpression durchgeführt wird.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Gebräuchliche Methoden zur Präparation der Proteingele und zur Identifizierung der Proteine sind die im 2-D Electrophoresis Handbook (GE Healthcare UK Ltd, England, Handbook 80-6429-60AC (2006)) und die von Hermann et al. (Electrophoresis, 22:1712-23 (2001))beschriebenen Vorgehensweisen. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden.

Ein Gen oder Allel ist chemisch ein Polynukleotid. Ein anderer Begriff hierfür ist Nukleinsäure, insbesondere Desoxyribonukleinsäure.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Gegenstand der Erfindung ist ein Verfahren zur Herstellung von organisch-chemischen Verbindungen durch Fermentation der erfindungsgemäßen rekombinanten Mikroorganismen der Familie Enterobacteriaceae, dadurch gekennzeichnet, dass man
a) die die gewünschte organisch-chemische Verbindung produzierenden Mikroorganismen, in denen man das mdtO-Gen oder für dessen Genprodukt kodierende Nukleotidsequenzen oder Allele verstärkt, insbesondere überexprimiert, in einem Medium kultiviert unter Bedingungen, bei denen die gewünschte organisch-chemische Verbindung im Medium oder in den Zellen angereichert wird, und,
b) die gewünschte organisch-chemische Verbindung isoliert, wobei gegebenenfalls weitere Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die insbesondere rekombinanten Mikroorganismen mit einem verstärkten oder überexprimierten mdtO-Gen, die ebenfalls Gegenstand der vorliegenden Erfindung sind, können organisch-chemische Verbindungen aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens oder ORFs verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Zur Herstellung der organisch-chemische Verbindungenanreichernden Stämme der Familie Enterbacteriaceae, welche ein verstärktes mdtO-Gen enthalten, werden bevorzugt Stämme verwendet (Ausgangsstämme bzw. Elternstämme), die bereits die Fähigkeit besitzen, die gewünschte organisch-chemische Verbindung in der Zelle anzureichern und/oder in das sie umgebende Nährmedium auszuscheiden bzw. in der Fermentationsbrühe zu akkumulieren. Hierfür wird in diesem Zusammenhang auch der Ausdruck "produzieren" verwendet. Insbesondere besitzen die für die Maßnahmen der Erfindung eingesetzten Stämme die Fähigkeit ≥ (mindestens) 0,25 g/l, ≥ 0,5 g/l, ≥ 1,0 g/l, ≥ 1,5 g/l, ≥ 2,0 g/l, ≥ 4 g/l der ≥ 10 g/l an organisch-chemischer Verbindung in ≤ (maximal) 120 Stunden, ≤ 96 Stunden, ≤ 48 Stunden, ≤ 36 Stunden, ≤ 24 Stunden oder ≤ 12 Stunden in der Zelle und/oder im Nährmedium bzw. der Fermentationsbrühe anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Die genannten organisch-chemische Verbindungenanreichernden Stämme produzieren zum Beispiel eine oder mehrere, bevorzugt eine oder im Wesentlichen eine Aminosäure ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin, bevorzugt ausgewählt aus der Gruppe L-Threonin, L-Tryptophan, L-Lysin, L-Valin, L-Isoleucin und L-Homoserin. Der Begriff L-Aminosäure oder Aminosäure umfasst auch deren Salze.

Der Begriff " eine oder im Wesentlichen eine Aminosäure" berücksichtigt, dass zusätzlich zu der gewünschten L-Aminosäure eine oder mehrer weitere der genannten L-Aminosäuren (Nebenaminosäuren) produziert werden können.

Der Anteil dieser Nebenaminosäuren beträgt ≥ 0 bis maximal 40%, bevorzugt ≥ 0 bis maximal 20%, besonders bevorzugt ≥ 0 bis maximal 10% und ganz besonders bevorzugt ≥ 0 bis maximal 5% bezogen auf die Menge der gewünschten L-Aminosäure.

Als zum Elternstamm geeignete, insbesondere L-Threonin produzierende bzw. ausscheidende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli TH 14.97 (WO 02/26993)
- Escherichia coli TH 21.97 (WO 02/26993)
- Escherichia coli BKIIM B-3996 (US-A-5,175,107)
- Escherichia coli BKIIM B-3996ΔtdhΔpckA/pVIC40 (WO 02/29080)
- Escherichia coli kat 13 (WO 98/04715)
- Escherichia coli Kat 69.9 (WO 02/26993)
- Escherichia coli KCCM-10132 (WO 00/09660)
- Escherichia coli KCCM-10168 (WO 01/14525)
- Escherichia coli KCCM-10133 (WO 00/09661)

Als zum Elternstamm geeignete L-Threonin produzierende bzw. ausscheidende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992))

L-Threonin produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der

Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Als zum Elternstamm geeignete, L-Tryptophan produzierende bzw. ausscheidende Stämme der Gattung Escherichia, insbesondere der Art Escherichia coli sind beispielsweise zu nennen:
- Escherichia coli JP4735/pMU3028 (US5,756,345)
- Escherichia coli JP6015/pMU91 (US5,756,345)
- Escherichia coli SV164(pGH5) (WO94/08031)
- E. coli AGX17(pGX44) (NRRL B-12263) (US4,371,614)
- E. coli AGX6(pGX50)aroP (NRRL B-12264) (US4,371,614)
- Escherichia coli AGX17/pGX50,pACKG4-pps (WO97/08333)
- Escherichia coli ATCC 31743 (CA1182409)
- E. coli C534/pD2310,pDM136 (ATCC 39795) (WO87/01130)
- Escherichia coli JB102/p5LRPS2 (US5,939,295).

L-Tryptophan produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phosphoenolpyruvat-Synthese, Verstärkung der D-Erythrose-4-Phosphat-Synthese, Verstärkung der 3-deoxy-D-arabino-heptulosonat-7-Phosphat(DHAP)-Synthese, Verstärkung der Chorismat-Biosynthese.

Als ein als Elternstamm geeigneter, L-Homoserin ausscheidender bzw. produzierender Stamm der Gattung Escherichia, insbesondere der Art Escherichia coli ist beispielsweise zu nennen:
Escherichia coli NZ10rhtA23/pAL4 (US 6,960,455).

L-Homoserin produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Bedürftigkeit für L-Threonin, Bedürftigkeit für L-Methionin, Bedürftigkeit für L-Isoleucin, eine defekte Homoserin-Kinase, gegebenenfalls Fähigkeit zur Saccharose-Verwertung, Verstärkung der Homoserin-Dehydrogenase I/Aspartatkinase I, bevorzugt der feed back resistenten Form, Verstärkung des RhtA-Genproduktes.

Als zum Elternstamm geeignete, L-Lysin ausscheidende bzw. produzierende Stämme der Gattung Escherichia, insbesondere der Art Escherichia coli sind beispielsweise zu nennen:
Escherichia coli pDA1/TOC21R (= CNCM 1-167) (FR-A-2511032)
Escherichia coli NRRL B-12199 (US4,346,170)
Escherichia coli NRRL B-12185 (US4,346,170).

Als zum Elternstamm geeignete, L-Isoleucin ausscheidende bzw. produzierende Stämme der Gattung Escherichia, insbesondere der Art Escherichia coli sind beispielsweise zu nennen:
Escherichia coli H-8670 (FERM BP-4051) (US5,460,958)
Escherichia coli H-8683 (FERM BP-4052) (US5,460,958)
Escherichia coli FERM BP-3628 (US5,362,637)
Escherichia coli FERM BP-3629 (US5,362,637)
Escherichia coli FERM BP-3630 (US5,362,637)
Escherichia coli H-9146 (FERM BP-5055) (US5,695,972)
Escherichia coli H-9156 (FERM BP-5056) (US5,695,972).

L-Isoleucin produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Resistenz gegen Isoleucin-Analoga wie Thiaisoleucin, Resistenz gegen Ethionin, Resistenz gegen Arginin-Hydroxamat, Resistenz gegen S-(2-Aminoethyl)-L-Cystein, Resistenz gegen D-Serin.

Als Elternstamm geeigneter, L-Valin ausscheidender bzw. produzierender Stamm der Gattung Escherichia, insbesondere der Art Escherichia coli ist beispielsweise zu nennen:
Escherichia coli AJ11502 (NRRL B-12288) (US-A- 4391907).

Bei den der Erfindung zugrunde liegenden Arbeiten wurde gefunden, dass Mikroorganismen der Familie Enterobacteriaceae nach Überexpression des Gens mdtO, oder dessen Allelen, vermehrt organisch-chemische Verbindungen, insbesondere L-Aminosäuren, insbesondere L-Threonin, L-Tryptophan, L-Lysin, L-Valin, L-Isoleucin und L-Homoserin, produzieren und in der Zelle oder im Medium anreichern.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminopeptidase) die N-terminale Aminosäure Methionin abgespalten werden kann.

Aus den ebenfalls zur Familie Enterobacteriaceae gehörenden Salmonella enterica subsp. arizonae serovar und Shigella flexneri ist die Nukleotidsequenz für das mdtO-Gen ebenso bekannt (Accession No.: CP000880 (REGION: 3290867-3292891 komplementär) und Accession No.: AE005674 (REGION: 4311053-4312954)). Weitere Nukleotidsequenzen für das mdtO-Gen findet man aus folgenden Enterobacteriaceae: Shigella boydii (Accession No.: CP000036) und Shigella sonnei (Accession No.: CP000038).

Das mdtO-Gen von Escherichia coli K12 wird unter anderem durch folgende Angaben beschrieben:
- Bezeichnung:: Translokase-Komponente des Multidrug-Transporters MdtNOP
- Funktion:: in der Cytoplasmamembran lokalisierter Transporter, der an der Vermittlung der multidrug resistance (MDR) beteiligt ist
- Referenz:: Sulavik et al., Antimicrobial Agents and Chemotherapy 45(4):1126-36 (2001)
- Accession No.:: NC_000913 (REGION: 4299050 - 4301101 (komplementär))

Das kodierte Polypeptid besitzt eine Länge von 683 Aminosäuren.
Alternativer Genname: ECK4074 , b4081 , yjcQ

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum mdtO-Gen von Escherichia coli unter der SEQ ID No. 1 und die bekannten Sequenzen zum mdtO-Gen von Salmonella enterica subsp. arizonae serovar und Shigella flexneri unter den SEQ ID No. 3 und SEQ ID No. 5 dargestellt. Die von diesen Leserahmen kodierten Proteine sind als SEQ ID No. 2, SEQ ID No. 4 und SEQ ID No. 6 dargestellt.

Die in den angegebenen Textstellen beschriebenen Gene oder offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des mdtO-Gens, welche funktionsneutrale Sinnmutationen enthalten, zählen unter anderem solche, die zu höchstens 15, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 8 in keinem Falle aber mehr als 10 Aminosäuren betreffen.

Zu den geeigneten Allelen gehören ferner solche, die durch Hybridisierung, insbesondere unter stringenten Bedingungen unter Verwendung von SEQ ID No. 1, SEQ ID No. 3 oder SEQ ID No. 5 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 75% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 75% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 75% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1, SEQ ID No. 3 oder SEQ ID No. 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Zur Erzielung einer Verstärkung, insbesondere einer Überexpression, können beispielsweise die Expression der Gene oder offenen Leserahmen oder Allele oder die katalytischen Eigenschaften des Proteins erhöht werden. Gegebenenfalls können beide Maßnahmen kombiniert werden.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens, bevorzugt des mdtO-Gens, befindet, mutiert werden. In gleicher Weise wirken Expressionskassetten oder Promotoren, die stromaufwärts des Strukturgens eingebaut werden. Durch induzierbare Promotoren ist es zusätzlich möglich, die Expression im Verlaufe der fermentativen L-Aminosäure-Produktion zu steigern. Des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene, ORFs oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)), die Verwendung dieser Plasmide ohne Induktion durch IPTG-Zugabe (= Isopropyl-β-D-Thiogalactopyranosid) führt zu einer sehr schwachen Expression von ohne eigenem Promotor klonierten Genen. Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Der Austausch des Promotors gegen einen zum Beispiel in genomweit-vergleichenden Expressionsanalysen ermittelten Promotor mit gleichmäßig hoher Expression im Verlauf eines Fermentationsprozesses bewirkt eine gleichmäßige Verstärkung. Die Veränderung einer Wachstumsphasen-abhängigen Genexpression kann beispielsweise wie bei Walker et al. (Journal of Bacteriology 181: 1269-80 (1999)) beschrieben durch die Verwendung des Wachstumsphasen-abhängigen fis Promotors oder einer der dort beschriebenen Mutationen des fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden. Desweiteren kann der Austausch eines Start-Codons zu dem in Escherichia coli mit 77% am häufigsten vorkommenden Codon ATG die Translation erheblich verbessern, da das Codon AUG zwei- bis dreimal effektiver ist als zum Beispiel die Codons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Codons kann optimiert werden, da zusammenwirkende Effekte zwischen dem Start-Codon und den flankierenden Bereichen beschrieben sind (Stenstrom et al., Gene 273(2):259-65 (2001); Hui et al., EMBO Journal 3(3):623-9 (1984)).

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolomé et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens das mdtO-Gen, oder für dessen Genprodukt kodierende Nukleotidsequenzen oder Allele trägt.

Unter dem Begriff Transformation versteht man die Aufnahme einer isolierten Nukleinsäure durch einen Wirt (Mikroorganismus).

Zur Erzeugung von mdtO-Allelen, die in den für das erfindungsgemäße Verfahren eingesetzten Mikroorganismen zum Einsatz kommen, können unter anderem im Stand der Technik beschriebene Methoden zur gerichteten Mutagenese verwendet werden.

Es können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Zur Konstruktion von Punktmutationen im mdtO-Gen kann zum Beispiel das Quick Change Site-Directed Mutagenesis Kit der Firma Stratagene (Amsterdam, Niederlande) verwendet werden. Bei Verwendung dieser Methoden wird das im Stand der Technik beschriebene mdtO-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden.

Die erzeugten mdtO-Allele können beispielsweise durch Transformation und das Verfahren des Gen- bzw. Allelaustausches in geeignete Stämme eingebaut werden.

Eine gebräuchliche Methode ist die von Hamilton et al. (Journal of Bacteriology 171, 4617 - 4622 (1989)) beschriebene Methode des Genaustauschs mit Hilfe eines konditional replizierenden pSC101-Derivates pMAK705 oder mit pK03 (Link et al., Journal of Bacteriology 179 : 6228-6237 (1997)). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Es ist ebenfalls möglich die erzeugten mdtO-Allele durch Konjugation oder Transduktion in verschiedene Stämme zu überführen.

Die Mutationen von mdtO können ebenfalls in Expressionsplasmiden durchgeführt werden, so dass die resultierenden Proteine überproduziert werden können.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Aminosäuren, insbesondere L-Threonin, mit Stämmen der Familie Enterobacteriaceae vorteilhaft sein, zusätzlich zur Verstärkung des mdtO-Gens, ein oder mehrere Enzym(e) der bekannten Aminosäure-Biosynthesewege oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

Ebenso kann es für die Produktion von L-Trytophan mit Stämmen der Familie Enterobacteriaceae vorteilhaft sein, zusätzlich zur Verstärkung des mdtO-Gens, ein oder mehrere Enzyme des bekannten Tryptophan-Biosyntheseweges oder Enzyme der Serin-Biosynthese oder Enzyme für die Produktion von 3-deoxy-D-arabino-heptulosonat-7-Phosphat (DHAP) und Chorismat zu verstärken. Die Verwendung endogener Gene wird im Allgemeinen bevorzugt.

So können beispielsweise für die Produktion von L-Threonin gleichzeitig eines oder mehrere der Gene, ausgewählt aus der Gruppe
mindestens ein Gen des für die Aspartatkinase, die Homoserin-Dehydrogenase, die Homoserinkinase und die Threoninsynthase kodierenden thrABC-Operons (US-A-4,278,765),
das für die Pyruvat-Carboxylase kodierende pyc-Gen von Corynebacterium glutamicum (WO 99/18228),
das für die Phosphoenolpyruvat-Synthase kodierende pps-Gen (Molecular and General Genetics 231(2): 332-336 (1992); WO 97/08333),
das für die Phosphoenolpyruvat-Carboxylase kodierende ppc-Gen (WO 02/064808),
die für die Untereinheiten der Pyridin-Transhydrogenase kodierenden Gene pntA und pntB (European Journal of Biochemistry 158: 647-653 (1986); WO 95/11985),
das Gen rhtC, welches für das Threoninresistenz vermittelnde Protein kodiert (EP-A-1 013 765),
das für das Threonin-Export-Carrier-Protein kodierende thrE-Gen von Corynebacterium glutamicum (WO 01/92545),
das für die Glutamat-Dehydrogenase kodierende gdhA-Gen (Nucleic Acids Research 11: 5257-5266 (1983); Gene 23: 199-209 (1983); DE19907347),
das für die Phosphohistidin-Protein-Hexose-Phosphotransferase des Phosphotransferase-Systems PTS kodierende ptsH-Gen des ptsHIcrr-Operons (WO 03/004674),
das für das Enzym I des Phosphotransferase-Systems PTS kodierende ptsI-Gen des ptsHIcrr-Operons (WO 03/004674),
das für die Glucose-spezifische IIA Komponente des Phosphotransferase-Systems PTS kodierende crr-Gen des ptsHIcrr-Operons (WO 03/004674),
das für die Glucose-spezifische IIBC Komponente kodierende ptsG-Gen (WO 03/004670),
das für die Cystein-Synthase A kodierende cysK-Gen (WO 03/006666),
das für den Regulator des cys-Regulons kodierende cysB-Gen (WO 03/006666),
das für das Flavoprotein der NADPH-Sulfit-Reduktase kodierende cysJ-Gen des cysJIH-Operons (WO 03/006666),
das für das Hämoprotein der NADPH-Sulfit-Reduktase kodierende cysI-Gen des cysJIH-Operons (WO 03/006666),
das für die Adenylylsulfat-Reduktase kodierende cysH-Gen des cysJIH-Operons (WO 03/006666),
das für die Decarboxylase Untereinheit der 2-Ketoglutarat Dehydrogenase kodierende sucA-Gen des sucABCD-Operons (WO 03/008614),
das für die Dihydrolipoyltranssuccinase E2 Untereinheit der 2-Ketoglutarat Dehydrogenase kodierende sucB-Gen des sucABCD-Operons (WO 03/008614),
das für die β-Untereinheit der Succinyl-CoA Synthetase kodierende sucC-Gen des suc ABCD-Operons (WO 03/008615),
das für die α-Untereinheit der Succinyl-CoA Synthetase kodierende sucD-Gen des sucABCD-Operons (WO 03/008615),
das Genprodukt des offenen Leserahmens (ORF) yjcG von Escherichia coli (Accession Number NC000913 (Region 4281276-4282925) des National Center for Biotechnology Information (NCBI, Bethesda, MD, USA), der besseren Übersichtlichkeit halber ist die Sequenz des yjcG-ORFs unter der SEQ ID No. 11 und die des von diesem Leserahmen kodierten Proteins als SEQ ID No. 12 dargestellt),
das Genprodukt des offenen Leserahmens (ORF) yibD von Escherichia coli (Accession Number AE000439 des National Center for Biotechnology Information (NCBI, Bethesda, MD, USA)), der besseren Übersichtlichkeit halber ist die Sequenz des yibD-ORFs unter der SEQ ID No. 13 und die des von diesem Leserahmen kodierten Proteins als SEQ ID No. 14 dargestellt),
das Genprodukt des offenen Leserahmens (ORF) yaaU von Escherichia coli (Accession Number NC000913 (Region 45807-47138) des National Center for Biotechnology Information (NCBI, Bethesda, MD, USA), der auch unter der Bezeichnung yaaU-ORF bekannt ist, der besseren Übersichtlichkeit halber ist die Sequenz des yaaU-ORFs unter der SEQ ID No. 15 und die des von diesem Leserahmen kodierten Proteins als SEQ ID No. 16 dargestellt),
das für ein Porin der äußeren Membran kodierende mdtP-Gen des mdtNOP-Operons (Accession Number NC000913 (Region 4,297,587 <- 4,299,053) des National Center for Biotechnology Information (NCBI, Bethesda, MD, USA),
das für ein Membranfusionsprotein kodierende mdtN-Gen des mdtNOP-Operons (Accession Number NC000913 (Region 4,301,101 <- 4,302,132) des National Center for Biotechnology Information (NCBI, Bethesda, MD, USA),
verstärkt, insbesondere überexprimiert werden.

Desweiteren können beispielsweise für die Produktion von L-Tryptophan, gleichzeitig eines oder mehrere der Gene, ausgewählt aus der Gruppe
mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons (Applied and Environmental Microbiology 38(2): 181-190 (1979)),
das für die 3-Phosphoglycerat-Dehydrogenase kodierende serA-Gen (WO87/01130)
das für die Phosphoserinphosphatase kodierende serB-Gen (WO87/01130)
das für die Phosphoserinaminotransferase kodierende serC-Gen (WO87/01130)
das für die L-Tyrosin sensitive DHAP-Synthase kodierende aroF-Gen (WO87/01130; EP1270721)
das für die L-Phenylalanin sensitive DHAP-Synthase kodierende aroG-Gen (WO87/01130; EP1270721)
das für die L-Tryptophan sensitive DHAP-Synthase kodierende aroH-Gen (WO87/01130; EP1270721)
das für die Phosphoenolpyruvat-Synthase kodierende pps-Gen (WO96/08567)
das für die Phosphoenolpyruvat-Carboxykinase kodierende pckA-Gen (W02004/090125),
das für die Transketolase A kodierende tktA-Gen (US5,168,056),
das für die Transketolase B kodierende tktB-Gen (US5,168,056),
das Genprodukt des offenen Leserahmens (ORF) yddG von Escherichia coli (Accession Number NC000913 (Region 1544312-1545193) des National Center for Biotechnology Information (NCBI, Bethesda, MD, USA)), der besseren Übersichtlichkeit halber ist die Sequenz des yddG-ORFs unter der SEQ ID No. 17 und die des von diesem Leserahmen kodierten Proteins als SEQ ID No. 18 dargestellt),
das für ein Porin der äußeren Membran kodierende mdtP-Gen des mdtNOP-Operons (Accession Number NC000913 (Region 4,297,587 <- 4,299,053) des National Center for Biotechnology Information (NCBI, Bethesda, MD, USA),
das für ein Membranfusionsprotein kodierende mdtN-Gen des mdtNOP-Operons (Accession Number NC000913 (Region 4,301,101 <- 4,302,132) des National Center for Biotechnology Information (NCBI, Bethesda, MD, USA),
verstärkt, insbesondere überexprimiert werden.

L-Threonin produzierende Mikroorganismen der Familie Enterobacteriaceae besitzen typischerweise eine "feed back" resistente bzw. desensibilisierte Aspartatkinase I/Homoserin-Dehydrogenase I. Unter "feed back" resistenter Aspartatkinase/Homoserin-Dehydrogenase versteht man Aspartatkinase/Homoserin-Dehydrogenase Enzyme (kodiert durch thrA, EC:2.7.2.4/ EC:1.1.1.3), die im Vergleich zur Wildform eine geringere Empfindlichkeit gegenüber der Hemmung durch Threonin oder Mischungen von Threonin und dem Threonin-Analogon α-Amino-β-Hydroxyvaleriansäure (AHV) oder AHV allein aufweisen. Die für diese desensibilisierten Enzyme kodierenden Gene bzw. Allele werden auch als thrA^{FBR}-Allele bezeichnet. Im Stand der Technik sind thrA^{FBR}-Allele beschrieben, die für Aspartatkinase/Homoserin-Dehydrogenase Varianten kodieren, welche Aminosäureaustausche im Vergleich zum Wildtypprotein besitzen. Die Kodierregion des Wildtyp thrA-Gens von Escherichia coli entsprechend der Zugangsnummer U00096.2 der NCBI Datenbank (Bethesda, MD, USA) ist in SEQ ID NO:7 und das von diesem Gen kodierte Polypeptid in SEQ ID NO:8 dargestellt.

Auch die Nukleotidsequenz des thrA-Gens von Serratia marcescens ist bekannt und unter der Zugangsnummer X60821 beim NCBI verfügbar. Die Kodierregion des Wildtyp thrA-Gens von Serratia marcescens ist in SEQ ID NO:9 und das von diesem Gen kodierte Polypeptid in SEQ ID NO:10 dargestellt.

Die für die Maßnahmen der Erfindung eingesetzten L-Threonin produzierenden Mikroorganismen der Familie Enterobacteriaceae verfügen bevorzugt über ein thrA-Allel, das für eine Aspartatkinase/Homoserin-Dehydrogenase Variante kodiert, welche die Aminosäuresequenz von SEQ ID NO:8 oder SEQ ID NO:10 besitzt, wobei diese eine oder mehrere der Aminosäureaustausche ausgewählt aus der Gruppe:
ThrA E253K (Austausch von L-Glutaminsäure an Position 253 des kodierten Enzyms Aspartatkinase/Homoserin-Dehydrogenase gemäß SEQ ID NO:8 oder SEQ ID NO:10 gegen L-Lysin; siehe Research Disclosure 505, 537 (2006)),
ThrA G330D (Austausch von Glycin an Position 330 des kodierten Enzyms Aspartatkinase/Homoserin-Dehydrogenase gemäß SEQ ID NO:8 oder SEQ ID NO:10 gegen L-Asparaginsäure; siehe Omori et al. (Journal of Bacteriology 175(3), 785-794 (1993)),
ThrA S345F (Austausch von L-Serin an Position 345 des kodierten Enzyms Aspartatkinase/Homoserin-Dehydrogenase gemäß SEQ ID NO:8 oder SEQ ID NO:10 gegen L-Phenylalanin; siehe Lee et al., Journal of Bacteriology 185(18): 5442-5451 (2003)),
ThrA S352, Austausch von L-Serin an Position 352 des kodierten Enzyms Aspartatkinase/Homoserin-Dehydrogenase gemäß SEQ ID NO:8 oder SEQ ID NO:10 gegen L-Phenylalanin, L-Tyrosin, L-Asparagin, L-Alanin, L-Arginin, L-Glutamin, L-Glutaminsäure, L-Histidin, L-Leucin, L-Methionin, L-Tryptophan oder L-Valin, bevorzugt gegen L-Phenylalanin; siehe Omori et al. (Journal of Bacteriology 175(3), 785-794 (1993) und Omori et al. (Journal of Bacteriology 175(4), 959-965 (1993),
ThrA A479T (Austausch von L-Alanin an Position 479 des kodierten Enzyms Aspartatkinase/Homoserin-Dehydrogenase gemäß SEQ ID NO:8 oder SEQ ID NO:10 gegen L-Threonin; siehe Omori et al. (Journal of Bacteriology 175(3), 785-794 (1993)),
umfasst.

Bevorzugt wird eines der thrA^{FBR}-Allele thrA E253K (Austausch von L-Glutaminsäure an Position 253 des kodierten Enzyms Aspartatkinase/Homoserin-Dehydrogenase gegen L-Lysin)oder S345F (Austausch von L-Serin an Position 345 des kodierten Enzyms Aspartatkinase/Homoserin-Dehydrogenase gegen L-Phenylalanin) gemäß SEQ ID NO:8.

Die hier beschriebenen thrA^{FBR}-Allele, die für ein Aspartatkinase/Homoserin-Dehydrogenase Enzym kodieren, können mit den oben beschriebenen Maßnahmen überexprimiert werden.

Gegebenenfalls kann es von Vorteil sein, nur eine gemäßigte Erhöhung der intrazellulären Aktivität oder Konzentration eines oder mehrerer Enzyme oder Proteine in einem Mikroorganismus durchzuführen, da eine zu starke Erhöhung zum Beispiel zu einer fehlerhaften Zellteilung oder veränderter Zellmorphologie bis hin zur Toxizität führen kann (Guthrie und Wickner, Journal of Bacteriology 172(10):5555-5562 (1990); Genevaux P. et al.; EMBO Reports 5(2): 195-200 (2004)).

Der Begriff "gemäßigte Erhöhung" beschreibt die Erhöhung der intrazellulären Aktivität oder Konzentration des entsprechenden Proteins um höchstens das 10-fache, das 8-fache, das 6-fache, das 4-fache, das 3-fache, das 2-fache oder das 1,5-fache bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm. Als nicht rekombinanter Mikroorganismus oder Elternstamm (parent strain) wird der Mikroorganismus verstanden, an dem die erfindungsgemäße Verstärkung oder Überexpression durchgeführt wird.

Weiterhin kann es für die Produktion von L-Aminosäuren, insbesondere L-Threonin, L-Tryptophan, L-Lysin, L-Valin, L-Isoleucin und L-Homoserin vorteilhaft sein, zusätzlich zur Verstärkung des mdtO-Gens, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

So können beispielsweise für die Produktion von L-Threonin gleichzeitig eines oder mehrere der Gene, ausgewählt aus der Gruppe
das für die Threonin-Dehydrogenase kodierende tdh-Gen (Journal of Bacteriology 169: 4716-4721 (1987)),
das für die Malat-Dehydrogenase (E.C. 1.1.1.37) kodierende mdh-Gen (Archives in Microbiology 149: 36-42 (1987)),
das für das Enzym Phosphoenolpyruvat-Carboxykinase kodierende pckA-Gen (WO 02/29080),
das für die Pyruvat-Oxidase kodierende poxB-Gen (WO 02/36797),
das für den DgsA-Regulator des Phosphotransferase-Systems kodierende dgsA-Gen (WO 02/081721), das auch unter der Bezeichnung mlc-Gen bekannt ist,
das für den Fructose-Repressor kodierende fruR-Gen (WO 02/081698), das auch unter der Bezeichnung cra-Gen bekannt ist,
das für den Sigma³⁸-Faktor kodierende rpoS-Gen (WO 01/05939), das auch unter der Bezeichnung katF-Gen bekannt ist,
das für die Aspartat Ammonium-Lyase kodierende aspA-Gen (WO 03/008603),
abgeschwächt, insbesondere ausgeschaltet oder die Expression verringert werden. Diese Massnahmen werden gegebenenfalls zusätzlich zu bzw. in geeigneter Kombination mit den angegebenen Massnahmen der Verstärkung von Genen zur Erhöhung der Threonin-Produktion durchgeführt.

Weiterhin kann es für die Produktion von L-Tryptophan vorteilhaft sein, zusätzlich zur Verstärkung des mdtO-Gens, eines oder mehrere der Gene ausgewählt aus der Gruppe
das für die Tryptophanase kodierende tnaA-Gen (US4,371,614),
das für den Repressor des trp-Operons kodierende trpR-Gen (US4,371,614)
das für die Chorismat-Mutase T und Prephenat-Dehydrogenase kodierende tyrA-Gen (US4,371,614),
das für die Chorismat-Mutase P und Prephenat-Dehydrogenase kodierende pheA-Gen (US4,371,614),
das für das Tryptophan spezifische Transportprotein kodierende mtr-Gen (US5,756,345),
das für die Tryptophan-Permease kodierende tnaB-Gen (US5,756,345),
das für den Transporter für aromatische Aminosäuren kodierende aroP-Gen (US5,756,345),
das für die L-Serin Deaminase kodierende sdaA-Gen (EP0149539),
das für die Glukose-6-Phosphat-Isomerase kodierende pgi-Gen (WO87/01130),
das für die Tyrosin-Aminotransferase kodierende tyrB-Gen (WO87/01130),
abzuschwächen, insbesondere auszuschalten oder die Expression zu verringern. Diese Massnahmen werden gegebenenfalls zusätzlich zu bzw. in geeigneter Kombination mit den angegebenen Massnahmen der Verstärkung von Genen zur Erhöhung der Tryptophan-Produktion durchgeführt.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Gegebenenfalls können die Maßnahmen zur Verstärkung und zur Abschwächung beliebig kombiniert werden.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben erhöht.

Erfindungsgemäß werden die hergestellten Mikroorganismen im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert. Zusammenfassungen über derartige Verfahren sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Bei einem batch-Verfahren werden, mit wenigen Ausnahmen, wie beispielsweise Sauerstoff und pH-Korrekturmittel, sämtliche Einsatzstoffe in Form eines Ansatzes vorgelegt und der Mikrorganismus in dem erhaltenen Medium kultiviert.

Bei einem fed batch-Verfahren wird der Mikrorganismus zunächst mittels eines batch-Verfahrens kultiviert (batch-Phase). Im Anschluss daran wird der Kultur ein für die Herstellung des Produktes wesentlicher Einsatzstoff, ggf. auch mehrere Einsatzstoffe, kontinuierlich oder diskontinuierlich hinzugefügt (Zulaufphase, feed-Phase). Im Falle der Herstellung von organisch-chemischen Verbindungen handelt es sich hierbei um eine Kohlenstoffquelle.

Bei einem repeated fed batch-Verfahren handelt es sich um ein fed batch-Verfahren, bei dem nach Abschluss der Fermentation ein Teil der erhaltenen Fermentationsbrühe als Inokulum zum Start eines erneuten repeated fed batch-Verfahrens dient. Dieser Zyklus kann ggf. mehrfach wiederholt werden. Repeated fed batch-Verfahren sind beispielsweise in der WO 02/18543 und WO 05/014843 beschrieben.

Bei einem kontinuierlichen Verfahren werden im Anschluß an ein batch- oder fed batch-Verfahren ein oder mehrere ggf. sämtliche Einsatzstoffe zu der Kultur kontinuierlich hinzugefügt und gleichzeitig Fermentationsbrühe entnommen. Kontinuierliche Verfahren sind beispielsweise in den Patentschriften US 5,763,230, WO 05/014840, WO 05/014841 und WO 05/014842 beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Im Allgemeinen enthält ein Kulturmedium unter anderem eine oder mehrere Kohlenstoffquelle(n), Stickstoffquelle(n) und Phosphorquelle(n).

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einzigen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung bzw. Akkumulation der L-Aminosäure in der Fermentations- bzw. Kulturbrühe. Die Kultur wird solange fortgesetzt, bis sich ein Maximum an der gewünschten L-Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Unter einer Fermentationsbrühe bzw. Kulturbrühe versteht man ein Fermentationsmedium, in dem ein Mikroorganismus für eine gewisse Zeit und bei einer gewissen Temperatur kultiviert wurde.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete L-Aminosäure, c) die im Laufe der Fermentation gebildeten organischen Nebenprodukte und

d) die durch die Fermentation nicht verbrauchten Bestandteile des/ der eingesetzten Fermentationsmediums/ Fermentationsmedien bzw. der Einsatzstoffe wie beispielsweise Vitamine wie Thiamin oder Salze wie Magnesiumsulfat.

Die hergestellte Kultur- bzw. Fermentationsbrühe kann anschließend gesammelt und die gewünschte L-Aminosäure bzw. das L-Aminosäure-haltige Produkt gewonnen oder isoliert werden.

In einer Verfahrensvariante wird die Fermentationsbrühe gegebenenfalls konzentriert und anschließend die L-Aminosäure gereinigt bzw. in reiner oder nahezu reiner Form isoliert. Typische Methoden zur Reinigung von L-Aminosäuren sind die Ionenaustauschchromatographie, die Kristallisation, Extraktionsverfahren und die Behandlung mit Aktivkohle. Hierdurch erhält man weitgehend reine L-Aminosäuren mit einem Gehalt von ≥ 90 Gew.-%, ≥ 95 Gew.-%, ≥ 96 Gew.-%, ≥ 97 Gew.-% ≥ 98 Gew.-% oder ≥ 99 Gew.-%.

Es ist ebenfalls möglich in einer anderen Verfahrensvariante aus der hergestellten Fermentationsbrühe ein Produkt herzustellen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von L-Aminosäuren zur Bestimmung der Konzentration zu einem oder mehreren Zeitpunkt(en) im Verlauf der Fermentation kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Das erfindungsgemäße Verfahren dient zur fermentativen Herstellung von organisch-chemischen Verbindungen, wie beispielsweise L-Aminosäuren, bevorzugt L-Threonin, L-Isoleucin, L-Valin, L-Methionin, L-Homoserin, L-Tryptophan und L-Lysin, insbesondere bevorzugt L-Threonin, L-Tryptophan, L-Lysin, L-Valin, L-Isoleucin und L-Homoserin.

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America 86: 2172-2175 (1989)) durchgeführt.

Die Bebrütungstemperatur bei der Herstellung von Stämmen und Transformanten ist 37°C.

### Beispiel 1

### Konstruktion des Expressionsplasmides pTrc99AmdtO

Das Gen mdtO aus E. coli K12 wurde unter Anwendung der Polymerase-Kettenreaktion (PCR) sowie synthetischer Oligonukleotiden amplifiziert. Ausgehend von der Nukleotidsequenz des mdtO-Gens in E. coli K12 MG1655 (Accession Number NC_000913 (REGION: 4299050 - 4301101 (komplementär)) wurden PCR-Primer synthetisiert (MWG Biotech, Ebersberg, Deutschland). Die Sequenzen der Primer wurden so modifiziert, dass Erkennungsstellen für Restriktionsenzyme entstanden. Für den mdtO1-Primer wurde die Erkennungssequenz für XbaI und für den mdtO2-Primer die Erkennungssequenz für HindIII gewählt, die in der unten dargestellten Nukleotidabfolge durch Unterstreichen markiert sind:
mdtO1: 5' - GGCCTCTAGA CGCTAATCTT GAGCCGCAAT - 3'
mdtO2: 5` - GGCCAAGCTT AGCAGACGTG AAAGTTGACG - 3`

Die für die PCR eingesetzte chromosomale E. coli K12 MG1655 DNA wurde nach Herstellerangaben mit "Qiagen Genomic-tips 100/G" (QIAGEN, Hilden, Deutschland) isoliert. Ein ca. 2120 bp großes DNA-Fragment konnte mit den spezifischen Primern unter Standard-PCR-Bedingungen (Innis et al. (1990) PCR Protocols. A Guide to Methods and Applications, Academic Press) mit der Vent-DNA-Polymerase (New England Biolabs, Frankfurt, Deutschland) amplifiziert werden.

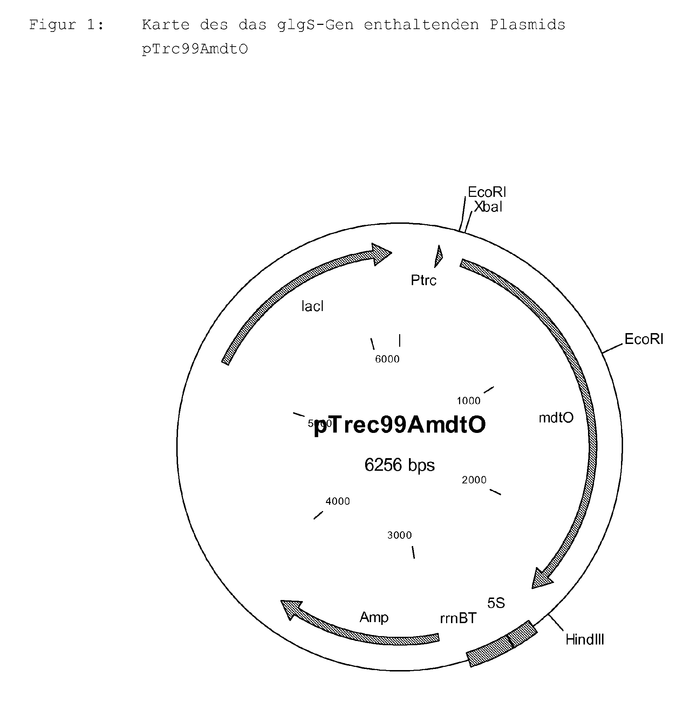

Das PCR-Produkt (die Aminosäuresequenz des korrespondierenden Proteins entspricht SEQ ID No. 2) wurde mit den Restriktionsenzymen XbaI und HindIII gespalten und mit dem Vektor pTrc99A (Pharmacia Biotech, Uppsala, Schweden), der mit den Enzymen XbaI und HindIII verdaut worden ist, ligiert. Der E. coli Stamm TOP10 (Invitrogen, Paisley, UK) wurde mit dem Ligationsansatz transformiert und Plasmid tragende Zellen auf LB Agar, der mit 50 µg/ml Ampicillin versetzt ist, selektioniert. Die erfolgreiche Klonierung konnte nach der Plasmid DNA Isolierung durch die Kontrollspaltung mit den Enzymen EcoRI, HindIII und XbaI nachgewiesen werden. Das Plasmid wird als pTrc99AmdtO (Figur 1) bezeichnet.

### Beispiel 2

### Herstellung von L-Threonin mit dem Stamm MG442/pTrc99AmdtO

Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 hinterlegt.

Der Stamm MG442 wurde mit dem in Beispiel 1 beschriebenen Expressionsplasmid pTrc99AmdtO und mit dem Vektor pTrc99A transformiert und auf LB Agar mit 50 µg/ml Ampicillin Plasmid tragende Zellen selektioniert. Auf diese Weise entstehen die Stämme MG442/pTrc99AmdtO und MG442/pTrc99A. Ausgewählte Einzelkolonien wurden anschließend auf Minimalmedium mit der folgenden Zusammensetzung: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar, 50 mg/l Ampicillin, weiter vermehrt. Die Bildung von L-Threonin wurde in batch Kulturen von 10 ml, die in 100 ml Erlenmeierkolben enthalten sind, überprüft. Dazu wurde 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose, 50 mg/l Ampicillin, beimpft und für 16 Stunden bei 37°C und 180 rpm auf einem ESR Inkubator der Firma Kühner AG (Birsfelden, Schweiz) inkubiert.

Je 250 µl dieser Vorkultur wurden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose, 50 mg/l Ampicillin) überimpft und für 48 Stunden bei 37°C inkubiert. Die Bildung von L-Threonin durch den Ausgangsstamm MG442 wird in der gleichen Weise überprüft, wobei jedoch keine Zugabe von Ampicillin zum Medium erfolgt. Nach der Inkubation wurde die optische Dichte (OD) der Kultursuspension mit einem LP2W-Photometer der Firma Dr. Lange (Düsseldorf, Deutschland) bei einer Messwellenlänge von 660 nm bestimmt.

Anschließend wurde die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

**Tabelle 1**

| Stamm | OD (660 nm) | L-Threonin g/l |
|---|---|---|
| MG442 | 5,5 | 1,4 |
| MG442/pTrc99A | 3,1 | 1,4 |
| MG442/pTrc99AmdtO | 5,6 | 2,0 |

### Kurze Beschreibung der Figur:

- Figur 1:: Karte des das mdtO-Gen enthaltenden Plasmids pTrc99AmdtO

Längenangaben sind als ca.-Angaben aufzufassen. Die verwendeten Abkürzungen und Bezeichnungen haben folgende Bedeutung:
Amp: Ampicillinresistenzgen
lacI: Gen für das Repressorprotein des trc-Promotors
Ptrc: trc-Promotorregion, IPTG-induzierbar
mdtO: Kodierregion des mdtO-Gens
5S: 5S rRNA-Region
rrnBT: rRNA-Terminator-Region

Die Abkürzungen für die Restriktionsenzyme haben folgende Bedeutung
EcoRI: Restriktionsendonuklease aus Escherichia coli
HindIII: Restriktionsendonuklease aus Haemophilus influenzae Rd
XbaI: Restriktionsendonuklease aus Xanthomonas campestris

## Patentansprüche

1. Rekombinante organisch-chemische Verbindungen produzierende Mikroorganismen der Familie Enterobacteriaceae, die ein verstärktes oder überexprimiertes mdtO-Gen enthalten, welches für die Translokase-Komponente eines Multidrug-Transporters (MDT) kodiert.

2. Mikroorganismen gemäss Anspruch 1, in denen ein dem mdtO-Gen entsprechendes Polynukleotid verstärkt wird, das für ein Polypeptid kodiert, dessen Aminosäuresequenz zu mindestens 80% identisch ist mit einer Aminosäuresequenz, ausgewählt aus der Gruppe SEQ ID No. 2, SEQ ID No. 4 und SEQ ID No. 6 .

3. Mikroorganismen gemäss Anspruch 2, **dadurch gekennzeichnet, dass** sie ein dem mdtO-Gen entsprechendes isoliertes Polynukleotid enthalten ausgewählt aus der Gruppe:
a) Polynukleotid mit einer Nukleotidsequenz, ausgewählt aus SEQ ID No. 1, SEQ ID No. 3, SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1, SEQ ID No. 3 oder SEQ ID No. 5 komplementären Sequenz unter stringenten Bedingungen hybridisiert, wobei die stringenten Bedingungen durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält,
wobei die Polynukleotide für die Translokase-Komponente eines Multidrug-Transporters (MDT) kodieren.

4. Mikroorganismen gemäss Anspruch 2, **dadurch gekennzeichnet, dass** das Polypeptid eine Aminosäuresequenz besitzt, die zu wenigstens 95% identisch ist mit einer der Sequenzen ausgewählt aus der Gruppe SEQ ID No. 2, SEQ ID No. 4 und SEQ ID No. 6.

5. Mikroorganismen gemäss Anspruch 2, **dadurch gekennzeichnet, dass** das Polypeptid die Aminosäuresequenz aufweist, die 100% identisch ist mit einer der Sequenzen SEQ ID No. 2 oder SEQ ID No. 4.

6. Mikroorganismus gemäss den Ansprüchen 1 bis 5, **dadurch gekennzeichnet, dass** sie durch Transformation, Transduktion, Konjugation oder eine Kombination dieser Methoden hergestellt werden, mit einem Vektor, der das mdtO-Gen, ein Allel dieses Gens oder Teile davon und/oder einen Promotor enthält.

7. Mikroorganismen gemäss den Ansprüchen 1 bis 6, in denen die Kopienzahl des mdtO-Gens oder der Allele um mindestens 1 erhöht vorliegt.

8. Mikroorganismen gemäss Anspruch 7, **dadurch gekennzeichnet, dass** die Erhöhung der Kopienzahl des mdtO-Gens um mindestens 1 durch Integration des Gens oder der Allele in das Chromosom der Mikroorganismen bewirkt wird.

9. Mikroorganismen gemäss Anspruch 7, **dadurch gekennzeichnet, dass** die Erhöhung der Kopienzahl des mdtO-Gens um mindestens 1 durch einen extrachromosomal replizierenden Vektor erzielt wird.

10. Mikroorganismen gemäss den Ansprüchen 1 bis 9, **dadurch gekennzeichnet, dass** man zur Erzielung der Verstärkung
a) die Promotor- und Regulationsregion oder die Ribosomenbindungsstelle stromaufwärts des mdtO-Gens mutiert, oder
b) Expressionskassetten oder Promotoren stromaufwärts des mdtO-Gens einbaut.

11. Mikroorganismen gemäss den Ansprüchen 1 bis 9, **dadurch gekennzeichnet, dass** die Expression des mdtO-Gens unter der Kontrolle eines die Expression des Gens verstärkenden Promotors steht.

12. Mikroorganismen gemäss den Ansprüchen 1 bis 11, **dadurch gekennzeichnet, dass** durch die Verstärkung des mdtO-Gens die Konzentration oder Aktivität des mdtO-Genproduktes (Proteins) um mindestens 10% erhöht sind, bezogen auf die Aktivität oder Konzentration des Genproduktes im für das mdtO-Gen nicht rekombinanten Mikroorganismus oder Elternstamm.

13. Mikroorganismen gemäss den Ansprüchen 1 bis 12, **dadurch gekennzeichnet, dass** die Mikroorganismen aus den Gattungen Escherichia, Erwinia, Providencia und Serratia ausgewählt sind.

14. Mikroorganismen gemäss Anspruch 13, **dadurch gekennzeichnet, dass** zusätzlich weitere Gene des Biosyntheseweges der gewünschten organisch-chemischen Verbindung verstärkt, insbesondere überexprimiert vorliegen.

15. Mikroorganismen gemäss den Ansprüchen 1 bis 14, **dadurch gekennzeichnet, dass** sie L-Threonin, L-Tryptophan, L-Lysin, L-Valin, L-Isoleucin oder L-Homoserin produzieren.

16. Verfahren zur Herstellung von organisch-chemischen Verbindungen durch Fermentation von rekombinanten Mikroorganismen der Familie Enterobacteriaceae, **dadurch gekennzeichnet, dass** man
a) die die gewünschten organisch-chemischen Verbindungen produzierenden Mikroorganismen gemäß den Ansprüchen 1 bis 15 in einem Medium kultiviert unter Bedingungen, bei denen die gewünschte Verbindung im Medium oder in den Zellen angereichert wird, und
b) die gewünschte Verbindung isoliert, wobei Bestandteile der Fermentationsbrühe und/oder die Biomasse in ihrer Gesamtheit oder Anteilen (≥ 0 bis 100%) im isolierten Produkt verbleiben oder vollständig entfernt werden.

17. Verfahren gemäss Anspruch 16 , **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pyridin-Transhydrogenase kodierenden Gene pntA und pntB,
f) das Gen rhtC, welches für das Threoninresistenz vermittelnde Protein kodiert,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die β-Untereinheit der Succinyl-CoA Synthetase kodierende sucC-Gen,
u) das für die α-Untereinheit der Succinyl-CoA Synthetase kodierende sucD-Gen,
v) das Genprodukt des offenen Leserahmens (ORF) yjcG von Escherichia coli,
w) das Genprodukt des offenen Leserahmens (ORF) yibD von Escherichia coli,
x) das Genprodukt des offenen Leserahmens (ORF) yaaU von Escherichia coli,
y) das für ein Porin der äußeren Membran kodierende mdtP-Gen, und
z) das für ein Membranfusionsprotein kodierende mdtN-Gen verstärkt, insbesondere überexprimiert.

18. Verfahren gemäss Anspruch 16, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
a) mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons,
b) das für die 3-Phosphoglycerat-Dehydrogenase kodierende serA-Gen,
c) das für die Phosphoserinphosphatase kodierende serB-Gen,
d) das für die Phosphoserinaminotransferase kodierende serC-Gen,
e) das für die L-Tyrosin sensitive DHAP-Synthase kodierende aroF-Gen,
f) das für die L-Phenylalanin sensitive DHAP-Synthase kodierende aroG-Gen,
g) das für die L-Tryptophan sensitive DHAP-Synthase kodierende aroH-Gen,
h) das für die Phosphoenolpyruvat-Synthase kodierende pps-Gen,
i) das für die Phosphoenolpyruvat-Carboxykinase kodierende pckA-Gen,
j) das für die Transketolase A kodierende tktA-Gen,
k) das für die Transketolase B kodierende tktB-Gen,
l) das Genprodukt des offenen Leserahmens (ORF) yddG von Escherichia coli,
m) das für ein Porin der äußeren Membran kodierende mdtP-Gen, und
n) das für ein Membranfusionsprotein kodierende mdtN-Gen verstärkt, insbesondere überexprimiert.

19. Verfahren gemäss Anspruch 19, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das für die Phosphoenolpyruvat-Carboxykinase kodierende pckA-Gen,
d) das für die Pyruvat-Oxidase kodierende poxB-Gen,
e) das für den DgsA-Regulator des Phosphotransferase-Systems kodierende dgsA-Gen,
f) das für den Fructose-Repressor kodierende fruR-Gen,
g) das für den Sigma³⁸-Faktor kodierende rpoS-Gen, und
h) das für die Aspartat Ammonium-Lyase kodierende aspA-Gen,
abschwächt, insbesondere ausschaltet oder die Expression verringert.

20. Verfahren gemäss den Ansprüchen 16 oder 18, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
a) das für die Tryptophanase kodierende tnaA-Gen,
b) das für den Repressor des trp-Operons kodierende trpR-Gen,
c) das für die Chorismat-Mutase T und Prephenat-Dehydrogenase kodierende tyrA-Gen,
d) das für die Chorismat-Mutase P und Prephenat-Dehydrogenase kodierende pheA-Gen,
e) das für das Tryptophan spezifische Transportprotein kodierende mtr-Gen,
f) das für die Tryptophan-Permease kodierende tnaB-Gen,
g) das für den Transporter für aromatische Aminosäuren kodierende aroP-Gen,
h) das für die L-Serin Deaminase kodierende sdaA-Gen,
i) das für die Glukose-6-Phosphat-Isomerase kodierende pgi-Gen, und
j) das für die Tyrosin-Aminotransferase kodierende tyrB-Gen,
abschwächt, insbesondere ausschaltet oder die Expression verringert.


SEQUENZPROTOKOLL

<110>  Evonik Degussa GmbH

<120>  Verfahren zur Herstellung von L-Aminosäuren unter Verwendung von
       verbesserten Stämmen der Familie Enterobacteriaceae

<130>  200800208

<160>  18

<170>  PatentIn version 3.3

<210>  1
<211>  2052
<212>  DNA
<213>  Escherichia coli


<220>
<221>  CDS
<222>  (1)..(2052)
<223>  mdtO Kodierregion

<400>  1
atg agc gcg ctc aac tcc ctg cca tta ccg gtg gtc agg ctg ctg gcg       48
Met Ser Ala Leu Asn Ser Leu Pro Leu Pro Val Val Arg Leu Leu Ala
1               5                   10                  15

ttc ttt cat gaa gag tta agc gag cgg cga cca ggt cgc gtg ccg cag       96
Phe Phe His Glu Glu Leu Ser Glu Arg Arg Pro Gly Arg Val Pro Gln
            20                  25                  30

acc gtg caa ctc tgg gta ggc tgc ctg ctg gtg att ctg atc tcg atg      144
Thr Val Gln Leu Trp Val Gly Cys Leu Leu Val Ile Leu Ile Ser Met
        35                  40                  45

acc ttt gag atc cct ttt gtg gcg tta tcg ctg gca gtg ctg ttt tac      192
Thr Phe Glu Ile Pro Phe Val Ala Leu Ser Leu Ala Val Leu Phe Tyr
    50                  55                  60

ggt att cag tcg aac gcg ttt tac acc aaa ttt gtc gcg atc ttg ttt      240
Gly Ile Gln Ser Asn Ala Phe Tyr Thr Lys Phe Val Ala Ile Leu Phe
65                  70                  75                  80

gtg gtt gcc acg gtg ctg gag atc ggc agc ctg ttt ttg atc tac aaa      288
Val Val Ala Thr Val Leu Glu Ile Gly Ser Leu Phe Leu Ile Tyr Lys
                85                  90                  95

tgg tca tac ggc gaa ccg ttg atc cga ttg atc atc gcc gga ccg atc      336
Trp Ser Tyr Gly Glu Pro Leu Ile Arg Leu Ile Ile Ala Gly Pro Ile
            100                 105                 110

ctg atg ggc tgc atg ttt ttg atg cgc acc cat cgc ttg ggg ctg gtc      384
Leu Met Gly Cys Met Phe Leu Met Arg Thr His Arg Leu Gly Leu Val
        115                 120                 125

ttt ttc gcc gtc gcc att gtc gct att tac ggg caa acc ttc ccc gcc      432
Phe Phe Ala Val Ala Ile Val Ala Ile Tyr Gly Gln Thr Phe Pro Ala
    130                 135                 140

atg ctc gac tat ccg gaa gtg gtc gtg cgc tta acg ctg tgg tgt atc      480
Met Leu Asp Tyr Pro Glu Val Val Val Arg Leu Thr Leu Trp Cys Ile
145                 150                 155                 160

gtt gtt ggc ctc tat cca acc ttg ctg atg acg tta atc ggc gtg ctg      528
Val Val Gly Leu Tyr Pro Thr Leu Leu Met Thr Leu Ile Gly Val Leu
                165                 170                 175

tgg ttt ccc agt cgt gcc att tcg caa atg cat cag gcg ctt aat gat     576
Trp Phe Pro Ser Arg Ala Ile Ser Gln Met His Gln Ala Leu Asn Asp
            180                 185                 190

cgg ctt gat gat gcc att agt cac ctg aca gac agc ctc gca ccg cta     624
Arg Leu Asp Asp Ala Ile Ser His Leu Thr Asp Ser Leu Ala Pro Leu
        195                 200                 205

ccc gaa acg cgg att gaa aga gag gcg ctg gcg cta caa aaa ctc aat     672
Pro Glu Thr Arg Ile Glu Arg Glu Ala Leu Ala Leu Gln Lys Leu Asn
    210                 215                 220

gtc ttt tgc ctc gcg gac gat gcc aac tgg cga act caa aac gca tgg     720
Val Phe Cys Leu Ala Asp Asp Ala Asn Trp Arg Thr Gln Asn Ala Trp
225                 230                 235                 240

tgg caa agc tgc gtg gca acg gta acc tac att tac tcg acg ctg aat     768
Trp Gln Ser Cys Val Ala Thr Val Thr Tyr Ile Tyr Ser Thr Leu Asn
                245                 250                 255

cgc tac gat ccc acc tct ttt gct gat tct cag gca att att gaa ttc     816
Arg Tyr Asp Pro Thr Ser Phe Ala Asp Ser Gln Ala Ile Ile Glu Phe
            260                 265                 270

cga caa aaa tta gct tca gaa atc aac aag ctg cag cat gcc gtt gct     864
Arg Gln Lys Leu Ala Ser Glu Ile Asn Lys Leu Gln His Ala Val Ala
        275                 280                 285

gaa ggt cag tgc tgg caa agc gac tgg cgg atc agt gaa agt gaa gcg     912
Glu Gly Gln Cys Trp Gln Ser Asp Trp Arg Ile Ser Glu Ser Glu Ala
    290                 295                 300

atg gcg gca cgg gaa tgt aac ctg gag aat atc tgc cag acg ttg tta     960
Met Ala Ala Arg Glu Cys Asn Leu Glu Asn Ile Cys Gln Thr Leu Leu
305                 310                 315                 320

caa ctg ggt cag atg gac ccg aat acg ccg cca acg ccc gca gcc aaa    1008
Gln Leu Gly Gln Met Asp Pro Asn Thr Pro Pro Thr Pro Ala Ala Lys
                325                 330                 335

ccg cca tca atg gcc gcc gat gct ttt acc aat cca gac tat atg cgc    1056
Pro Pro Ser Met Ala Ala Asp Ala Phe Thr Asn Pro Asp Tyr Met Arg
            340                 345                 350

tac gcg gta aaa acg ctg ctc gcc tgt ttg atc tgt tac acc ttt tac    1104
Tyr Ala Val Lys Thr Leu Leu Ala Cys Leu Ile Cys Tyr Thr Phe Tyr
        355                 360                 365

agc ggc gtg gac tgg gaa ggc att cac acc tgt atg ctg aca tgc gtg    1152
Ser Gly Val Asp Trp Glu Gly Ile His Thr Cys Met Leu Thr Cys Val
    370                 375                 380

atc gtc gct aac cca aat gtc ggt tcg tcg tac cag aag atg gtg ctg    1200
Ile Val Ala Asn Pro Asn Val Gly Ser Ser Tyr Gln Lys Met Val Leu
385                 390                 395                 400

cgt ttt ggc ggg gcc ttt tgc ggc gcg att ctg gcg ctg tta ttc acg    1248
Arg Phe Gly Gly Ala Phe Cys Gly Ala Ile Leu Ala Leu Leu Phe Thr
                405                 410                 415

cta ctg gtc atg ccc tgg ctg gac aat att gtc gaa ttg ctg ttt gtg    1296
Leu Leu Val Met Pro Trp Leu Asp Asn Ile Val Glu Leu Leu Phe Val
            420                 425                 430

ctg gca ccg att ttc ctg ttg ggc gca tgg att gcc acc agc tct gaa    1344
Leu Ala Pro Ile Phe Leu Leu Gly Ala Trp Ile Ala Thr Ser Ser Glu
        435                 440                 445

cgc tct tct tat atc ggc aca cag atg gtg gtc acc ttc gcg ctc gcc    1392

Arg Ser Ser Tyr Ile Gly Thr Gln Met Val Val Thr Phe Ala Leu Ala
    450                 455                 460

acg ctc gaa aac gtt ttt ggc cca gtg tac gac ctg gtg gaa att cgc      1440
Thr Leu Glu Asn Val Phe Gly Pro Val Tyr Asp Leu Val Glu Ile Arg
465                 470                 475                 480

gat cgc gcc ctg ggt atc atc att ggt acc gtg gtg tcc gcg gtg att      1488
Asp Arg Ala Leu Gly Ile Ile Ile Gly Thr Val Val Ser Ala Val Ile
                485                 490                 495

tac acc ttt gtc tgg cct gaa agt gaa gcg cgc aca ctg ccg caa aaa      1536
Tyr Thr Phe Val Trp Pro Glu Ser Glu Ala Arg Thr Leu Pro Gln Lys
            500                 505                 510

ctg gct ggc acg ctg ggt atg tta agt aaa gta atg cgg atc cca cgc      1584
Leu Ala Gly Thr Leu Gly Met Leu Ser Lys Val Met Arg Ile Pro Arg
        515                 520                 525

cag cag gaa gtc acg gct ctg cgc act tat ctg caa att cgt atc ggt      1632
Gln Gln Glu Val Thr Ala Leu Arg Thr Tyr Leu Gln Ile Arg Ile Gly
    530                 535                 540

ctg cat gcg gcg ttt aat gcc tgt gaa gag atg tgc caa cgc gtg gcg      1680
Leu His Ala Ala Phe Asn Ala Cys Glu Glu Met Cys Gln Arg Val Ala
545                 550                 555                 560

ctg gag cgt caa ctg gac agc gaa gaa cgc gca tta ctg att gaa cgt      1728
Leu Glu Arg Gln Leu Asp Ser Glu Glu Arg Ala Leu Leu Ile Glu Arg
                565                 570                 575

tcg caa acg gtt att cgt cag ggc cgc gat ctt ctt cac gcc tgg gat      1776
Ser Gln Thr Val Ile Arg Gln Gly Arg Asp Leu Leu His Ala Trp Asp
            580                 585                 590

gcc acc tgg aac tcg gcg cag gcg ctg gat aac gca cta cag ccg gac      1824
Ala Thr Trp Asn Ser Ala Gln Ala Leu Asp Asn Ala Leu Gln Pro Asp
        595                 600                 605

aga gca ggt cag ttt gcc gac gcc ctg gag aaa tac gct gcc ggt ctg      1872
Arg Ala Gly Gln Phe Ala Asp Ala Leu Glu Lys Tyr Ala Ala Gly Leu
    610                 615                 620

gca acc gca ctc agc cgt tct cct caa ata acg ctt gaa gag aca ccc      1920
Ala Thr Ala Leu Ser Arg Ser Pro Gln Ile Thr Leu Glu Glu Thr Pro
625                 630                 635                 640

gcc tct cag gcc atc ctg ccc acc tta tta aaa cag gag caa cac gtc      1968
Ala Ser Gln Ala Ile Leu Pro Thr Leu Leu Lys Gln Glu Gln His Val
                645                 650                 655

tgc cag ctt ttc gcc cgc ttg cca gac tgg aca gcc ccg gca tta acg      2016
Cys Gln Leu Phe Ala Arg Leu Pro Asp Trp Thr Ala Pro Ala Leu Thr
            660                 665                 670

ccc gcc acg gaa cag gca caa gga gcc acg caa tga                      2052
Pro Ala Thr Glu Gln Ala Gln Gly Ala Thr Gln
        675                 680

<210>  2
<211>  683
<212>  PRT
<213>  Escherichia coli

<400>  2

Met Ser Ala Leu Asn Ser Leu Pro Leu Pro Val Val Arg Leu Leu Ala
1               5                   10                  15

Phe Phe His Glu Glu Leu Ser Glu Arg Arg Pro Gly Arg Val Pro Gln
            20                  25                  30

Thr Val Gln Leu Trp Val Gly Cys Leu Leu Val Ile Leu Ile Ser Met
        35                  40                  45

Thr Phe Glu Ile Pro Phe Val Ala Leu Ser Leu Ala Val Leu Phe Tyr
    50                  55                  60

Gly Ile Gln Ser Asn Ala Phe Tyr Thr Lys Phe Val Ala Ile Leu Phe
65                  70                  75                  80

Val Val Ala Thr Val Leu Glu Ile Gly Ser Leu Phe Leu Ile Tyr Lys
                85                  90                  95

Trp Ser Tyr Gly Glu Pro Leu Ile Arg Leu Ile Ile Ala Gly Pro Ile
            100                 105                 110

Leu Met Gly Cys Met Phe Leu Met Arg Thr His Arg Leu Gly Leu Val
        115                 120                 125

Phe Phe Ala Val Ala Ile Val Ala Ile Tyr Gly Gln Thr Phe Pro Ala
    130                 135                 140

Met Leu Asp Tyr Pro Glu Val Val Val Arg Leu Thr Leu Trp Cys Ile
145                 150                 155                 160

Val Val Gly Leu Tyr Pro Thr Leu Leu Met Thr Leu Ile Gly Val Leu
                165                 170                 175

Trp Phe Pro Ser Arg Ala Ile Ser Gln Met His Gln Ala Leu Asn Asp
            180                 185                 190

Arg Leu Asp Asp Ala Ile Ser His Leu Thr Asp Ser Leu Ala Pro Leu
        195                 200                 205

Pro Glu Thr Arg Ile Glu Arg Glu Ala Leu Ala Leu Gln Lys Leu Asn
    210                 215                 220

Val Phe Cys Leu Ala Asp Asp Ala Asn Trp Arg Thr Gln Asn Ala Trp
225                 230                 235                 240

Trp Gln Ser Cys Val Ala Thr Val Thr Tyr Ile Tyr Ser Thr Leu Asn
                245                 250                 255

Arg Tyr Asp Pro Thr Ser Phe Ala Asp Ser Gln Ala Ile Ile Glu Phe
            260                 265                 270

Arg Gln Lys Leu Ala Ser Glu Ile Asn Lys Leu Gln His Ala Val Ala
        275                 280                 285

Glu Gly Gln Cys Trp Gln Ser Asp Trp Arg Ile Ser Glu Ser Glu Ala
    290                 295                 300

Met Ala Ala Arg Glu Cys Asn Leu Glu Asn Ile Cys Gln Thr Leu Leu
305                 310                 315                 320

Gln Leu Gly Gln Met Asp Pro Asn Thr Pro Pro Thr Pro Ala Ala Lys
                325                 330                 335

Pro Pro Ser Met Ala Ala Asp Ala Phe Thr Asn Pro Asp Tyr Met Arg
            340                 345                 350

Tyr Ala Val Lys Thr Leu Leu Ala Cys Leu Ile Cys Tyr Thr Phe Tyr
        355                 360                 365

Ser Gly Val Asp Trp Glu Gly Ile His Thr Cys Met Leu Thr Cys Val
    370                 375                 380

Ile Val Ala Asn Pro Asn Val Gly Ser Ser Tyr Gln Lys Met Val Leu
385                 390                 395                 400

Arg Phe Gly Gly Ala Phe Cys Gly Ala Ile Leu Ala Leu Leu Phe Thr
                405                 410                 415

Leu Leu Val Met Pro Trp Leu Asp Asn Ile Val Glu Leu Leu Phe Val
            420                 425                 430

Leu Ala Pro Ile Phe Leu Leu Gly Ala Trp Ile Ala Thr Ser Ser Glu
        435                 440                 445

Arg Ser Ser Tyr Ile Gly Thr Gln Met Val Val Thr Phe Ala Leu Ala
    450                 455                 460

Thr Leu Glu Asn Val Phe Gly Pro Val Tyr Asp Leu Val Glu Ile Arg
465                 470                 475                 480

Asp Arg Ala Leu Gly Ile Ile Ile Gly Thr Val Val Ser Ala Val Ile
                485                 490                 495

Tyr Thr Phe Val Trp Pro Glu Ser Glu Ala Arg Thr Leu Pro Gln Lys
            500                 505                 510

Leu Ala Gly Thr Leu Gly Met Leu Ser Lys Val Met Arg Ile Pro Arg
        515                 520                 525

Gln Gln Glu Val Thr Ala Leu Arg Thr Tyr Leu Gln Ile Arg Ile Gly
    530                 535                 540

Leu His Ala Ala Phe Asn Ala Cys Glu Glu Met Cys Gln Arg Val Ala
545                 550                 555                 560

Leu Glu Arg Gln Leu Asp Ser Glu Glu Arg Ala Leu Leu Ile Glu Arg
                565                 570                 575

Ser Gln Thr Val Ile Arg Gln Gly Arg Asp Leu Leu His Ala Trp Asp
            580                 585                 590

Ala Thr Trp Asn Ser Ala Gln Ala Leu Asp Asn Ala Leu Gln Pro Asp
        595                 600                 605

Arg Ala Gly Gln Phe Ala Asp Ala Leu Glu Lys Tyr Ala Ala Gly Leu
    610                 615                 620

Ala Thr Ala Leu Ser Arg Ser Pro Gln Ile Thr Leu Glu Glu Thr Pro
625                 630                 635                 640

Ala Ser Gln Ala Ile Leu Pro Thr Leu Leu Lys Gln Glu Gln His Val
                645                 650                 655

Cys Gln Leu Phe Ala Arg Leu Pro Asp Trp Thr Ala Pro Ala Leu Thr
            660                 665                 670

Pro Ala Thr Glu Gln Ala Gln Gly Ala Thr Gln
        675                 680

<210>  3
<211>  2025
<212>  DNA
<213>  Salmonella enterica

<220>
<221>  CDS
<222>  (1)..(2025)
<223>  mdtO Kodierregion

<400>  3
atg aac gcg ctt aat tac ctg ccg cta tcg gtc gtt aaa ttg ctg gct       48
Met Asn Ala Leu Asn Tyr Leu Pro Leu Ser Val Val Lys Leu Leu Ala
1               5                   10                  15

ttt ttt cat gag gag ctt agt gaa cag cgc cct ggc cga ctc aca cag       96
Phe Phe His Glu Glu Leu Ser Glu Gln Arg Pro Gly Arg Leu Thr Gln
            20                  25                  30

gta ggt cag ttg tgg atc ggt tgt cta ctg gtc gta ctg atc tct atg      144
Val Gly Gln Leu Trp Ile Gly Cys Leu Leu Val Val Leu Ile Ser Met
        35                  40                  45

acc ttt gag atc ccg ttt ctg gct atc tct ctg gca gtg ttg ttt tat      192
Thr Phe Glu Ile Pro Phe Leu Ala Ile Ser Leu Ala Val Leu Phe Tyr
    50                  55                  60

ggc gta cag tcg aac gcg ttt tac acc aaa ttc gtg gca atc ctg ttt      240
Gly Val Gln Ser Asn Ala Phe Tyr Thr Lys Phe Val Ala Ile Leu Phe
65                  70                  75                  80

gtg gtc gcg acg gtg ctg gaa atc ggc agt ctg ttt ctg atc tac aaa      288
Val Val Ala Thr Val Leu Glu Ile Gly Ser Leu Phe Leu Ile Tyr Lys
                85                  90                  95

tgg tcc tat agc tat ccg cta atg cgc atg att atc gcc agc gcg gta      336

Trp Ser Tyr Ser Tyr Pro Leu Met Arg Met Ile Ile Ala Ser Ala Val
            100                 105                 110

gtg ctg gtt tgc atg ttt atg atg cgt acc cac cgt ctg ggg ctg ctc      384
Val Leu Val Cys Met Phe Met Met Arg Thr His Arg Leu Gly Leu Leu
        115                 120                 125

ttt ttc gct gtg gct atc gtc gcc gta tac ggg caa acc ttc ccc gga      432
Phe Phe Ala Val Ala Ile Val Ala Val Tyr Gly Gln Thr Phe Pro Gly
    130                 135                 140

atg ctc gac tat ccg gag gta gtg gtt cgt ctc acg ctg tgg tgt atc      480
Met Leu Asp Tyr Pro Glu Val Val Val Arg Leu Thr Leu Trp Cys Ile
145                 150                 155                 160

gtt gtt gga ctc tac cca acg ctg ctg atg gta ctg atc ggc gtg ctg      528
Val Val Gly Leu Tyr Pro Thr Leu Leu Met Val Leu Ile Gly Val Leu
                165                 170                 175

tgg ttt ccc agc cgc gcg gta act caa atg cgg cac gcg ttg tgt gcc      576
Trp Phe Pro Ser Arg Ala Val Thr Gln Met Arg His Ala Leu Cys Ala
            180                 185                 190

cgg cta gac gac gcc gtt agc cat ttg ttg agc gac gtc gcg ccg caa      624
Arg Leu Asp Asp Ala Val Ser His Leu Leu Ser Asp Val Ala Pro Gln
        195                 200                 205

gcg gaa aaa tac att gag cgg aac gtg ctg gcg cta caa aag cta aat      672
Ala Glu Lys Tyr Ile Glu Arg Asn Val Leu Ala Leu Gln Lys Leu Asn
    210                 215                 220

gta ttt tgc ctg gcc gat gat gcc gac tgg cag gcg cgt agc acg tgg      720
Val Phe Cys Leu Ala Asp Asp Ala Asp Trp Gln Ala Arg Ser Thr Trp
225                 230                 235                 240

tgg caa aac tgc gtt tcc acc gtc act tat ctg tac act acg ctc aac      768
Trp Gln Asn Cys Val Ser Thr Val Thr Tyr Leu Tyr Thr Thr Leu Asn
                245                 250                 255

cgt tat tct gcc gac gcc gcg tcg caa tat ccg gcg ctg gtg caa aag      816
Arg Tyr Ser Ala Asp Ala Ala Ser Gln Tyr Pro Ala Leu Val Gln Lys
            260                 265                 270

ata cga aat gag atc gct tcg ctg caa cag gcg gtc gcc cag ggc gaa      864
Ile Arg Asn Glu Ile Ala Ser Leu Gln Gln Ala Val Ala Gln Gly Glu
        275                 280                 285

ccc tgg cag agt aca tgg tcg ctc agc gcg caa gag cgc gtc gcc gcc      912
Pro Trp Gln Ser Thr Trp Ser Leu Ser Ala Gln Glu Arg Val Ala Ala
    290                 295                 300

cgg gcg tgc ggg ctg gaa act atc tgc gac caa ctg cgc cag ctt gga      960
Arg Ala Cys Gly Leu Glu Thr Ile Cys Asp Gln Leu Arg Gln Leu Gly
305                 310                 315                 320

gag atg tcg ccc gat acg ccg cct gcg cct gcc gcc agg ccc ccc gcc     1008
Glu Met Ser Pro Asp Thr Pro Pro Ala Pro Ala Ala Arg Pro Pro Ala
                325                 330                 335

atg acg ccg gat gcc ttc agt aac ccg gtc tac ata cgc tac gcg ctg     1056
Met Thr Pro Asp Ala Phe Ser Asn Pro Val Tyr Ile Arg Tyr Ala Leu
            340                 345                 350

aaa acg ctg ctg gcc tgc atg att tgc tac gtc ttc tat tcc ggc gtc     1104
Lys Thr Leu Leu Ala Cys Met Ile Cys Tyr Val Phe Tyr Ser Gly Val
        355                 360                 365

gac tgg gaa ggc att cac acc tgt atg ctg aca tgt att atc gtg gcc     1152
Asp Trp Glu Gly Ile His Thr Cys Met Leu Thr Cys Ile Ile Val Ala

370                 375                 380

aat cca agc atc ggt tcg tca tat cag aaa atg tcg cta cgc ttt ggc      1200
Asn Pro Ser Ile Gly Ser Ser Tyr Gln Lys Met Ser Leu Arg Phe Gly
385                 390                 395                 400

ggc gcg ctt tgc ggc gcg cta ctg gcg ctg ctg gtc gcc att ttc gcc      1248
Gly Ala Leu Cys Gly Ala Leu Leu Ala Leu Leu Val Ala Ile Phe Ala
                405                 410                 415

atg ccc tgg ctg gat aac att gcc gaa ctg ctg tgc ctg cta gcg cct      1296
Met Pro Trp Leu Asp Asn Ile Ala Glu Leu Leu Cys Leu Leu Ala Pro
            420                 425                 430

gtc ttc ctg ctc ggc gcc tgg ata gcc aca gga tcg gaa cga tcg tcc      1344
Val Phe Leu Leu Gly Ala Trp Ile Ala Thr Gly Ser Glu Arg Ser Ser
        435                 440                 445

tat atc ggc aca caa atg att gtc acc ttt gcc ctg gcg acg ctc gaa      1392
Tyr Ile Gly Thr Gln Met Ile Val Thr Phe Ala Leu Ala Thr Leu Glu
    450                 455                 460

aac gtc ttt ggc ccg gtt tac gat ctg gta gaa att cgc gat cgc gcg      1440
Asn Val Phe Gly Pro Val Tyr Asp Leu Val Glu Ile Arg Asp Arg Ala
465                 470                 475                 480

atg ggg att tta atc ggc act gca gtt tcc gcc gtc att tat acg ttt      1488
Met Gly Ile Leu Ile Gly Thr Ala Val Ser Ala Val Ile Tyr Thr Phe
                485                 490                 495

atc tgg ccg gaa agc gaa gcg gac act ctg ccg caa aaa ctg gct ggc      1536
Ile Trp Pro Glu Ser Glu Ala Asp Thr Leu Pro Gln Lys Leu Ala Gly
            500                 505                 510

gcg tta ggc atc ctg ggc aag cta ttg cgt atc ccg cgc cag cct gac      1584
Ala Leu Gly Ile Leu Gly Lys Leu Leu Arg Ile Pro Arg Gln Pro Asp
        515                 520                 525

cct acg tcg caa cgg aca tac ctt cag cta cga att ggt tgc cat gcg      1632
Pro Thr Ser Gln Arg Thr Tyr Leu Gln Leu Arg Ile Gly Cys His Ala
    530                 535                 540

gca ttc aat gcc tgt gaa gag atg ggc gaa cgc gtc gcg ctg gaa tac      1680
Ala Phe Asn Ala Cys Glu Glu Met Gly Glu Arg Val Ala Leu Glu Tyr
545                 550                 555                 560

caa ctt agc gcc aaa gaa cgc gcg cgt ctg ctc gcc gac agc cag gcc      1728
Gln Leu Ser Ala Lys Glu Arg Ala Arg Leu Leu Ala Asp Ser Gln Ala
                565                 570                 575

gtc atc gat ctg gga cgt gaa ata ctc tat cgc tgg gat acg acc tgg      1776
Val Ile Asp Leu Gly Arg Glu Ile Leu Tyr Arg Trp Asp Thr Thr Trp
            580                 585                 590

aat agc gct ctc cct tcg gat cac gac gcg cag caa acg cac gct gag      1824
Asn Ser Ala Leu Pro Ser Asp His Asp Ala Gln Gln Thr His Ala Glu
        595                 600                 605

cgc att gcg caa gca tta gaa cat tat gct gcc ggc ctt gcg agc cgt      1872
Arg Ile Ala Gln Ala Leu Glu His Tyr Ala Ala Gly Leu Ala Ser Arg
    610                 615                 620

acc agc cgc gcg cct gcg ctc gat ttg acg gtg tcg ccc aca tta cct      1920
Thr Ser Arg Ala Pro Ala Leu Asp Leu Thr Val Ser Pro Thr Leu Pro
625                 630                 635                 640

gaa cag gaa caa ggt atc gtg cgc ctg atg tct tgc tta cca gac tgg      1968
Glu Gln Glu Gln Gly Ile Val Arg Leu Met Ser Cys Leu Pro Asp Trp
                645                 650                 655

acg gcc ccg gaa cac acg cca gcc gaa act ccg cag caa gga act acc      2016
Thr Ala Pro Glu His Thr Pro Ala Glu Thr Pro Gln Gln Gly Thr Thr
            660                 665                 670

cca tta tga                                                         2025
Pro Leu

<210>  4
<211>  674
<212>  PRT
<213>  Salmonella enterica

<400>  4

Met Asn Ala Leu Asn Tyr Leu Pro Leu Ser Val Val Lys Leu Leu Ala
1               5                   10                  15

Phe Phe His Glu Glu Leu Ser Glu Gln Arg Pro Gly Arg Leu Thr Gln
            20                  25                  30

Val Gly Gln Leu Trp Ile Gly Cys Leu Leu Val Val Leu Ile Ser Met
        35                  40                  45

Thr Phe Glu Ile Pro Phe Leu Ala Ile Ser Leu Ala Val Leu Phe Tyr
    50                  55                  60

Gly Val Gln Ser Asn Ala Phe Tyr Thr Lys Phe Val Ala Ile Leu Phe
65                  70                  75                  80

Val Val Ala Thr Val Leu Glu Ile Gly Ser Leu Phe Leu Ile Tyr Lys
                85                  90                  95

Trp Ser Tyr Ser Tyr Pro Leu Met Arg Met Ile Ile Ala Ser Ala Val
            100                 105                 110

Val Leu Val Cys Met Phe Met Met Arg Thr His Arg Leu Gly Leu Leu
        115                 120                 125

Phe Phe Ala Val Ala Ile Val Ala Val Tyr Gly Gln Thr Phe Pro Gly
    130                 135                 140

Met Leu Asp Tyr Pro Glu Val Val Val Arg Leu Thr Leu Trp Cys Ile
145                 150                 155                 160

Val Val Gly Leu Tyr Pro Thr Leu Leu Met Val Leu Ile Gly Val Leu
                165                 170                 175

Trp Phe Pro Ser Arg Ala Val Thr Gln Met Arg His Ala Leu Cys Ala
            180                 185                 190

Arg Leu Asp Asp Ala Val Ser His Leu Leu Ser Asp Val Ala Pro Gln
        195                 200                 205

Ala Glu Lys Tyr Ile Glu Arg Asn Val Leu Ala Leu Gln Lys Leu Asn
    210                 215                 220

Val Phe Cys Leu Ala Asp Asp Ala Asp Trp Gln Ala Arg Ser Thr Trp
225                 230                 235                 240

Trp Gln Asn Cys Val Ser Thr Val Thr Tyr Leu Tyr Thr Thr Leu Asn
                245                 250                 255

Arg Tyr Ser Ala Asp Ala Ala Ser Gln Tyr Pro Ala Leu Val Gln Lys
            260                 265                 270

Ile Arg Asn Glu Ile Ala Ser Leu Gln Gln Ala Val Ala Gln Gly Glu
        275                 280                 285

Pro Trp Gln Ser Thr Trp Ser Leu Ser Ala Gln Glu Arg Val Ala Ala
    290                 295                 300

Arg Ala Cys Gly Leu Glu Thr Ile Cys Asp Gln Leu Arg Gln Leu Gly
305                 310                 315                 320

Glu Met Ser Pro Asp Thr Pro Pro Ala Pro Ala Ala Arg Pro Pro Ala
                325                 330                 335

Met Thr Pro Asp Ala Phe Ser Asn Pro Val Tyr Ile Arg Tyr Ala Leu
            340                 345                 350

Lys Thr Leu Leu Ala Cys Met Ile Cys Tyr Val Phe Tyr Ser Gly Val
        355                 360                 365

Asp Trp Glu Gly Ile His Thr Cys Met Leu Thr Cys Ile Ile Val Ala
    370                 375                 380

Asn Pro Ser Ile Gly Ser Ser Tyr Gln Lys Met Ser Leu Arg Phe Gly
385                 390                 395                 400

Gly Ala Leu Cys Gly Ala Leu Leu Ala Leu Leu Val Ala Ile Phe Ala
                405                 410                 415

Met Pro Trp Leu Asp Asn Ile Ala Glu Leu Leu Cys Leu Leu Ala Pro
            420                 425                 430

Val Phe Leu Leu Gly Ala Trp Ile Ala Thr Gly Ser Glu Arg Ser Ser
        435                 440                 445

Tyr Ile Gly Thr Gln Met Ile Val Thr Phe Ala Leu Ala Thr Leu Glu
    450                 455                 460

Asn Val Phe Gly Pro Val Tyr Asp Leu Val Glu Ile Arg Asp Arg Ala
465                 470                 475                 480

Met Gly Ile Leu Ile Gly Thr Ala Val Ser Ala Val Ile Tyr Thr Phe

                485                 490                 495

Ile Trp Pro Glu Ser Glu Ala Asp Thr Leu Pro Gln Lys Leu Ala Gly
            500                 505                 510

Ala Leu Gly Ile Leu Gly Lys Leu Leu Arg Ile Pro Arg Gln Pro Asp
        515                 520                 525

Pro Thr Ser Gln Arg Thr Tyr Leu Gln Leu Arg Ile Gly Cys His Ala
    530                 535                 540

Ala Phe Asn Ala Cys Glu Glu Met Gly Glu Arg Val Ala Leu Glu Tyr
545                 550                 555                 560

Gln Leu Ser Ala Lys Glu Arg Ala Arg Leu Leu Ala Asp Ser Gln Ala
                565                 570                 575

Val Ile Asp Leu Gly Arg Glu Ile Leu Tyr Arg Trp Asp Thr Thr Trp
            580                 585                 590

Asn Ser Ala Leu Pro Ser Asp His Asp Ala Gln Gln Thr His Ala Glu
        595                 600                 605

Arg Ile Ala Gln Ala Leu Glu His Tyr Ala Ala Gly Leu Ala Ser Arg
    610                 615                 620

Thr Ser Arg Ala Pro Ala Leu Asp Leu Thr Val Ser Pro Thr Leu Pro
625                 630                 635                 640

Glu Gln Glu Gln Gly Ile Val Arg Leu Met Ser Cys Leu Pro Asp Trp
                645                 650                 655

Thr Ala Pro Glu His Thr Pro Ala Glu Thr Pro Gln Gln Gly Thr Thr
            660                 665                 670

Pro Leu

<210> 5
<211> 1902
<212> DNA
<213> Shigella flexneri

<220>
<221> CDS
<222> (1)..(1902)
<223> mdtO Kodierregion

<400> 5
atg act ttt gag atc cct ttt gtg gcg tta tcg ctg gca gtg ctg ttt 48
Met Thr Phe Glu Ile Pro Phe Val Ala Leu Ser Leu Ala Val Leu Phe
1               5                   10                  15

tac ggt att cag tcg aac gcg ttt tac acc aaa ttt gtg gcg atc ttg 96
Tyr Gly Ile Gln Ser Asn Ala Phe Tyr Thr Lys Phe Val Ala Ile Leu

20                  25                  30

ttt gtg gtt gcc acg gtg ctg gag atc ggc agc ctg ttt ttg atc tac       144
Phe Val Val Ala Thr Val Leu Glu Ile Gly Ser Leu Phe Leu Ile Tyr
        35                  40                  45

aaa tgg tca tac ggc gaa ccg ttg atc cga ttg atc atc gcc ggg ccg       192
Lys Trp Ser Tyr Gly Glu Pro Leu Ile Arg Leu Ile Ile Ala Gly Pro
    50                  55                  60

atc ctg atg ggc tgc atg ttt ttg atg cgc acc cat cgg ctg gga ctg       240
Ile Leu Met Gly Cys Met Phe Leu Met Arg Thr His Arg Leu Gly Leu
65                  70                  75                  80

gtc ttt ttc gct gtc gcc att tac ggg caa acc ttc ccc gcc atg ctc       288
Val Phe Phe Ala Val Ala Ile Tyr Gly Gln Thr Phe Pro Ala Met Leu
                85                  90                  95

gac tat ccg gaa gta gtc gtg cgc tta acg ctg tgg tgt atc gtt gtt       336
Asp Tyr Pro Glu Val Val Val Arg Leu Thr Leu Trp Cys Ile Val Val
            100                 105                 110

ggc ctc tat cca acc ttg ctg atg acg tta atc ggc gtg ctg tgg ttt       384
Gly Leu Tyr Pro Thr Leu Leu Met Thr Leu Ile Gly Val Leu Trp Phe
        115                 120                 125

ccc aat cgt gcc att acg caa atg cat caa gcg ctt aat gat cgg ctt       432
Pro Asn Arg Ala Ile Thr Gln Met His Gln Ala Leu Asn Asp Arg Leu
    130                 135                 140

gat gat gcc att agc cac ctg acg gac agc ctc gca ccg cta ccc gaa       480
Asp Asp Ala Ile Ser His Leu Thr Asp Ser Leu Ala Pro Leu Pro Glu
145                 150                 155                 160

acg cgg att gaa aga gag gcg ctg gcg ctg caa aaa ctg aat gtc ttt       528
Thr Arg Ile Glu Arg Glu Ala Leu Ala Leu Gln Lys Leu Asn Val Phe
                165                 170                 175

tgt ctc gcg gac gat gcc aac tgg cga act caa agc gca tgg tgg caa       576
Cys Leu Ala Asp Asp Ala Asn Trp Arg Thr Gln Ser Ala Trp Trp Gln
            180                 185                 190

agc tgc gtg gca acg gta acc tac att tac tcg acg ctg aat cgc tac       624
Ser Cys Val Ala Thr Val Thr Tyr Ile Tyr Ser Thr Leu Asn Arg Tyr
        195                 200                 205

gat ccc acc tct ttt gct gat tct cag gca att att gaa ttt cgg caa       672
Asp Pro Thr Ser Phe Ala Asp Ser Gln Ala Ile Ile Glu Phe Arg Gln
    210                 215                 220

aaa tta gcc tca gaa atc aac aag ctg cag cat gcc gtt gct gaa ggt       720
Lys Leu Ala Ser Glu Ile Asn Lys Leu Gln His Ala Val Ala Glu Gly
225                 230                 235                 240

cag tgc tgg caa agc gac tgg cgg ctc agt gaa agt gaa gcg gtg gcg       768
Gln Cys Trp Gln Ser Asp Trp Arg Leu Ser Glu Ser Glu Ala Val Ala
                245                 250                 255

gca cgg gaa tgt aac ctg gag aat atc tgc cag acg ttg tta caa ctg       816
Ala Arg Glu Cys Asn Leu Glu Asn Ile Cys Gln Thr Leu Leu Gln Leu
            260                 265                 270

ggc cag atg aac ccg aat acg ccg cca acg ccc gcc gcc aaa ccg cca       864
Gly Gln Met Asn Pro Asn Thr Pro Pro Thr Pro Ala Ala Lys Pro Pro
        275                 280                 285

tca atg gtc gcc gat gct ttt acc aat cca gac tat ata cgc tac gcg       912
Ser Met Val Ala Asp Ala Phe Thr Asn Pro Asp Tyr Ile Arg Tyr Ala
    290                 295                 300

gta aaa aca ttg ctc gcc tgt ttg atc tgt tac acc ttt tac agc ggc      960
Val Lys Thr Leu Leu Ala Cys Leu Ile Cys Tyr Thr Phe Tyr Ser Gly
305                 310                 315                 320

gtg gac tgg gaa ggc att cac acc tgt atg ctg acc tgc gtg atc gtc      1008
Val Asp Trp Glu Gly Ile His Thr Cys Met Leu Thr Cys Val Ile Val
                325                 330                 335

gct aat cca aat gtc ggt tcg tcg tac cag aag atg gtg ctg cgt ttt      1056
Ala Asn Pro Asn Val Gly Ser Ser Tyr Gln Lys Met Val Leu Arg Phe
            340                 345                 350

ggc ggg gcc ttt tgc ggc gcg att ctg gcg ctg tta ttc acg cta ctg      1104
Gly Gly Ala Phe Cys Gly Ala Ile Leu Ala Leu Leu Phe Thr Leu Leu
        355                 360                 365

gtc atg ccc tgg ctg gac aat att gtc gaa ttg ctg ttt gtg ctg gca      1152
Val Met Pro Trp Leu Asp Asn Ile Val Glu Leu Leu Phe Val Leu Ala
    370                 375                 380

ccg att ttc ctg ttg ggc gca tgg att gcc acc agc tct gaa cgc tct      1200
Pro Ile Phe Leu Leu Gly Ala Trp Ile Ala Thr Ser Ser Glu Arg Ser
385                 390                 395                 400

tct tat atc ggc aca cag atg gtg gtc acc ttc gcg ctc gcc acg ctc      1248
Ser Tyr Ile Gly Thr Gln Met Val Val Thr Phe Ala Leu Ala Thr Leu
                405                 410                 415

gaa aac gtt ttt ggc cca gtg tac gac ctg gtg gaa att cgc gat cgc      1296
Glu Asn Val Phe Gly Pro Val Tyr Asp Leu Val Glu Ile Arg Asp Arg
            420                 425                 430

gcc ctg ggt atc atc att ggt acc gtg gtg tcc gcg atg att tac acc      1344
Ala Leu Gly Ile Ile Ile Gly Thr Val Val Ser Ala Met Ile Tyr Thr
        435                 440                 445

ttt gtc tgg cct gaa agt gaa gcg cgc acg ctg ccg caa aaa ctg gct      1392
Phe Val Trp Pro Glu Ser Glu Ala Arg Thr Leu Pro Gln Lys Leu Ala
    450                 455                 460

ggc gcg ctg ggt atg tta agt aaa gta atg cgg atc cca cgc cag cag      1440
Gly Ala Leu Gly Met Leu Ser Lys Val Met Arg Ile Pro Arg Gln Gln
465                 470                 475                 480

gaa gtc acg gct ctg cgc act tat ctg caa att cgt atc ggt ctg cat      1488
Glu Val Thr Ala Leu Arg Thr Tyr Leu Gln Ile Arg Ile Gly Leu His
                485                 490                 495

gcg gcg ttt aat gcc tgt gaa gag atg tgc caa cgc gtg gcg ctg gag      1536
Ala Ala Phe Asn Ala Cys Glu Glu Met Cys Gln Arg Val Ala Leu Glu
            500                 505                 510

cgt caa ctg gac agc gaa gaa cgc gcc tta ctg att gaa cgt tcg caa      1584
Arg Gln Leu Asp Ser Glu Glu Arg Ala Leu Leu Ile Glu Arg Ser Gln
        515                 520                 525

acg gtt att cgt cag ggc cgc gat atc ctt cac gcc tgg gat gcc acc      1632
Thr Val Ile Arg Gln Gly Arg Asp Ile Leu His Ala Trp Asp Ala Thr
    530                 535                 540

tgg aat tcg gcg cag gcg ctg gat aac gca cta cag ccg gac aga gca      1680
Trp Asn Ser Ala Gln Ala Leu Asp Asn Ala Leu Gln Pro Asp Arg Ala
545                 550                 555                 560

ggt cag ttt gcc gac gcc ctg gag aaa tac gct gcc ggt ctg gca acc      1728
Gly Gln Phe Ala Asp Ala Leu Glu Lys Tyr Ala Ala Gly Leu Ala Thr
                565                 570                 575

gca ctc agc cgt tct cct caa ata acg ctt gaa gag aca ccc acc tcg     1776
Ala Leu Ser Arg Ser Pro Gln Ile Thr Leu Glu Glu Thr Pro Thr Ser
            580                 585                 590

cag gcc atc ctg ccc acc tta tta aaa cag gag caa cac gtc tgc cag     1824
Gln Ala Ile Leu Pro Thr Leu Leu Lys Gln Glu Gln His Val Cys Gln
        595                 600                 605

ctt ttc gcc cgc ttg cca gac tgg aca gcc ccg gca tta acg ccc gcc     1872
Leu Phe Ala Arg Leu Pro Asp Trp Thr Ala Pro Ala Leu Thr Pro Ala
    610                 615                 620

acg gaa cag gca caa gga gcc acg caa tga                             1902
Thr Glu Gln Ala Gln Gly Ala Thr Gln
625                 630


<210>  6
<211>  633
<212>  PRT
<213>  Shigella flexneri

<400>  6

Met Thr Phe Glu Ile Pro Phe Val Ala Leu Ser Leu Ala Val Leu Phe
1               5                   10                  15


Tyr Gly Ile Gln Ser Asn Ala Phe Tyr Thr Lys Phe Val Ala Ile Leu
            20                  25                  30


Phe Val Val Ala Thr Val Leu Glu Ile Gly Ser Leu Phe Leu Ile Tyr
        35                  40                  45


Lys Trp Ser Tyr Gly Glu Pro Leu Ile Arg Leu Ile Ile Ala Gly Pro
    50                  55                  60


Ile Leu Met Gly Cys Met Phe Leu Met Arg Thr His Arg Leu Gly Leu
65                  70                  75                  80


Val Phe Phe Ala Val Ala Ile Tyr Gly Gln Thr Phe Pro Ala Met Leu
                85                  90                  95


Asp Tyr Pro Glu Val Val Val Arg Leu Thr Leu Trp Cys Ile Val Val
            100                 105                 110


Gly Leu Tyr Pro Thr Leu Leu Met Thr Leu Ile Gly Val Leu Trp Phe
        115                 120                 125


Pro Asn Arg Ala Ile Thr Gln Met His Gln Ala Leu Asn Asp Arg Leu
    130                 135                 140


Asp Asp Ala Ile Ser His Leu Thr Asp Ser Leu Ala Pro Leu Pro Glu
145                 150                 155                 160


Thr Arg Ile Glu Arg Glu Ala Leu Ala Leu Gln Lys Leu Asn Val Phe
                165                 170                 175


Cys Leu Ala Asp Asp Ala Asn Trp Arg Thr Gln Ser Ala Trp Trp Gln

180                 185                 190

Ser Cys Val Ala Thr Val Thr Tyr Ile Tyr Ser Thr Leu Asn Arg Tyr
        195                 200                 205

Asp Pro Thr Ser Phe Ala Asp Ser Gln Ala Ile Ile Glu Phe Arg Gln
    210                 215                 220

Lys Leu Ala Ser Glu Ile Asn Lys Leu Gln His Ala Val Ala Glu Gly
225                 230                 235                 240

Gln Cys Trp Gln Ser Asp Trp Arg Leu Ser Glu Ser Glu Ala Val Ala
                245                 250                 255

Ala Arg Glu Cys Asn Leu Glu Asn Ile Cys Gln Thr Leu Leu Gln Leu
            260                 265                 270

Gly Gln Met Asn Pro Asn Thr Pro Pro Thr Pro Ala Ala Lys Pro Pro
        275                 280                 285

Ser Met Val Ala Asp Ala Phe Thr Asn Pro Asp Tyr Ile Arg Tyr Ala
    290                 295                 300

Val Lys Thr Leu Leu Ala Cys Leu Ile Cys Tyr Thr Phe Tyr Ser Gly
305                 310                 315                 320

Val Asp Trp Glu Gly Ile His Thr Cys Met Leu Thr Cys Val Ile Val
                325                 330                 335

Ala Asn Pro Asn Val Gly Ser Ser Tyr Gln Lys Met Val Leu Arg Phe
            340                 345                 350

Gly Gly Ala Phe Cys Gly Ala Ile Leu Ala Leu Leu Phe Thr Leu Leu
        355                 360                 365

Val Met Pro Trp Leu Asp Asn Ile Val Glu Leu Leu Phe Val Leu Ala
    370                 375                 380

Pro Ile Phe Leu Leu Gly Ala Trp Ile Ala Thr Ser Ser Glu Arg Ser
385                 390                 395                 400

Ser Tyr Ile Gly Thr Gln Met Val Val Thr Phe Ala Leu Ala Thr Leu
                405                 410                 415

Glu Asn Val Phe Gly Pro Val Tyr Asp Leu Val Glu Ile Arg Asp Arg
            420                 425                 430

Ala Leu Gly Ile Ile Ile Gly Thr Val Val Ser Ala Met Ile Tyr Thr
        435                 440                 445

Phe Val Trp Pro Glu Ser Glu Ala Arg Thr Leu Pro Gln Lys Leu Ala
    450                 455                 460

Gly Ala Leu Gly Met Leu Ser Lys Val Met Arg Ile Pro Arg Gln Gln
465                 470                 475                 480

Glu Val Thr Ala Leu Arg Thr Tyr Leu Gln Ile Arg Ile Gly Leu His
                485                 490                 495

Ala Ala Phe Asn Ala Cys Glu Glu Met Cys Gln Arg Val Ala Leu Glu
            500                 505                 510

Arg Gln Leu Asp Ser Glu Glu Arg Ala Leu Leu Ile Glu Arg Ser Gln
        515                 520                 525

Thr Val Ile Arg Gln Gly Arg Asp Ile Leu His Ala Trp Asp Ala Thr
    530                 535                 540

Trp Asn Ser Ala Gln Ala Leu Asp Asn Ala Leu Gln Pro Asp Arg Ala
545                 550                 555                 560

Gly Gln Phe Ala Asp Ala Leu Glu Lys Tyr Ala Ala Gly Leu Ala Thr
                565                 570                 575

Ala Leu Ser Arg Ser Pro Gln Ile Thr Leu Glu Glu Thr Pro Thr Ser
            580                 585                 590

Gln Ala Ile Leu Pro Thr Leu Leu Lys Gln Glu Gln His Val Cys Gln
        595                 600                 605

Leu Phe Ala Arg Leu Pro Asp Trp Thr Ala Pro Ala Leu Thr Pro Ala
    610                 615                 620

Thr Glu Gln Ala Gln Gly Ala Thr Gln
625                 630

<210>  7
<211>  2463
<212>  DNA
<213>  Escherichia coli

<220>
<221>  CDS
<222>  (1)..(2463)
<223>  thrA-Kodierregion

<400>  7
atg cga gtg ttg aag ttc ggc ggt aca tca gtg gca aat gca gaa cgt       48
Met Arg Val Leu Lys Phe Gly Gly Thr Ser Val Ala Asn Ala Glu Arg
1               5                   10                  15

ttt ctg cgt gtt gcc gat att ctg gaa agc aat gcc agg cag ggg cag       96
Phe Leu Arg Val Ala Asp Ile Leu Glu Ser Asn Ala Arg Gln Gly Gln
            20                  25                  30

gtg gcc acc gtc ctc tct gcc ccc gcc aaa atc acc aac cac ctg gtg      144
Val Ala Thr Val Leu Ser Ala Pro Ala Lys Ile Thr Asn His Leu Val
        35                  40                  45

gcg atg att gaa aaa acc att agc ggc cag gat gct tta ccc aat atc     192
Ala Met Ile Glu Lys Thr Ile Ser Gly Gln Asp Ala Leu Pro Asn Ile
    50                  55                  60

agc gat gcc gaa cgt att ttt gcc gaa ctt ttg acg gga ctc gcc gcc     240
Ser Asp Ala Glu Arg Ile Phe Ala Glu Leu Leu Thr Gly Leu Ala Ala
65                  70                  75                  80

gcc cag ccg ggg ttc ccg ctg gcg caa ttg aaa act ttc gtc gat cag     288
Ala Gln Pro Gly Phe Pro Leu Ala Gln Leu Lys Thr Phe Val Asp Gln
                85                  90                  95

gaa ttt gcc caa ata aaa cat gtc ctg cat ggc att agt ttg ttg ggg     336
Glu Phe Ala Gln Ile Lys His Val Leu His Gly Ile Ser Leu Leu Gly
            100                 105                 110

cag tgc ccg gat agc atc aac gct gcg ctg att tgc cgt ggc gag aaa     384
Gln Cys Pro Asp Ser Ile Asn Ala Ala Leu Ile Cys Arg Gly Glu Lys
        115                 120                 125

atg tcg atc gcc att atg gcc ggc gta tta gaa gcg cgc ggt cac aac     432
Met Ser Ile Ala Ile Met Ala Gly Val Leu Glu Ala Arg Gly His Asn
    130                 135                 140

gtt act gtt atc gat ccg gtc gaa aaa ctg ctg gca gtg ggg cat tac     480
Val Thr Val Ile Asp Pro Val Glu Lys Leu Leu Ala Val Gly His Tyr
145                 150                 155                 160

ctc gaa tct acc gtc gat att gct gag tcc acc cgc cgt att gcg gca     528
Leu Glu Ser Thr Val Asp Ile Ala Glu Ser Thr Arg Arg Ile Ala Ala
                165                 170                 175

agc cgc att ccg gct gat cac atg gtg ctg atg gca ggt ttc acc gcc     576
Ser Arg Ile Pro Ala Asp His Met Val Leu Met Ala Gly Phe Thr Ala
            180                 185                 190

ggt aat gaa aaa ggc gaa ctg gtg gtg ctt gga cgc aac ggt tcc gac     624
Gly Asn Glu Lys Gly Glu Leu Val Val Leu Gly Arg Asn Gly Ser Asp
        195                 200                 205

tac tct gct gcg gtg ctg gct gcc tgt tta cgc gcc gat tgt tgc gag     672
Tyr Ser Ala Ala Val Leu Ala Ala Cys Leu Arg Ala Asp Cys Cys Glu
    210                 215                 220

att tgg acg gac gtt gac ggg gtc tat acc tgc gac ccg cgt cag gtg     720
Ile Trp Thr Asp Val Asp Gly Val Tyr Thr Cys Asp Pro Arg Gln Val
225                 230                 235                 240

ccc gat gcg agg ttg ttg aag tcg atg tcc tac cag gaa gcg atg gag     768
Pro Asp Ala Arg Leu Leu Lys Ser Met Ser Tyr Gln Glu Ala Met Glu
                245                 250                 255

ctt tcc tac ttc ggc gct aaa gtt ctt cac ccc cgc acc att acc ccc     816
Leu Ser Tyr Phe Gly Ala Lys Val Leu His Pro Arg Thr Ile Thr Pro
            260                 265                 270

atc gcc cag ttc cag atc cct tgc ctg att aaa aat acc gga aat cct     864
Ile Ala Gln Phe Gln Ile Pro Cys Leu Ile Lys Asn Thr Gly Asn Pro
        275                 280                 285

caa gca cca ggt acg ctc att ggt gcc agc cgt gat gaa gac gaa tta     912
Gln Ala Pro Gly Thr Leu Ile Gly Ala Ser Arg Asp Glu Asp Glu Leu
    290                 295                 300

ccg gtc aag ggc att tcc aat ctg aat aac atg gca atg ttc agc gtt     960
Pro Val Lys Gly Ile Ser Asn Leu Asn Asn Met Ala Met Phe Ser Val
305                 310                 315                 320

tct ggt ccg ggg atg aaa ggg atg gtc ggc atg gcg gcg cgc gtc ttt     1008
Ser Gly Pro Gly Met Lys Gly Met Val Gly Met Ala Ala Arg Val Phe
                325                 330                 335

gca gcg atg tca cgc gcc cgt att tcc gtg gtg ctg att acg caa tca     1056
Ala Ala Met Ser Arg Ala Arg Ile Ser Val Val Leu Ile Thr Gln Ser
            340                 345                 350

tct tcc gaa tac agc atc agt ttc tgc gtt cca caa agc gac tgt gtg     1104
Ser Ser Glu Tyr Ser Ile Ser Phe Cys Val Pro Gln Ser Asp Cys Val
        355                 360                 365

cga gct gaa cgg gca atg cag gaa gag ttc tac ctg gaa ctg aaa gaa     1152
Arg Ala Glu Arg Ala Met Gln Glu Glu Phe Tyr Leu Glu Leu Lys Glu
    370                 375                 380

ggc tta ctg gag ccg ctg gca gtg acg gaa cgg ctg gcc att atc tcg     1200
Gly Leu Leu Glu Pro Leu Ala Val Thr Glu Arg Leu Ala Ile Ile Ser
385                 390                 395                 400

gtg gta ggt gat ggt atg cgc acc ttg cgt ggg atc tcg gcg aaa ttc     1248
Val Val Gly Asp Gly Met Arg Thr Leu Arg Gly Ile Ser Ala Lys Phe
                405                 410                 415

ttt gcc gca ctg gcc cgc gcc aat atc aac att gtc gcc att gct cag     1296
Phe Ala Ala Leu Ala Arg Ala Asn Ile Asn Ile Val Ala Ile Ala Gln
            420                 425                 430

gga tct tct gaa cgc tca atc tct gtc gtg gta aat aac gat gat gcg     1344
Gly Ser Ser Glu Arg Ser Ile Ser Val Val Val Asn Asn Asp Asp Ala
        435                 440                 445

acc act ggc gtg cgc gtt act cat cag atg ctg ttc aat acc gat cag     1392
Thr Thr Gly Val Arg Val Thr His Gln Met Leu Phe Asn Thr Asp Gln
    450                 455                 460

gtt atc gaa gtg ttt gtg att ggc gtc ggt ggc gtt ggc ggt gcg ctg     1440
Val Ile Glu Val Phe Val Ile Gly Val Gly Gly Val Gly Gly Ala Leu
465                 470                 475                 480

ctg gag caa ctg aag cgt cag caa agc tgg ctg aag aat aaa cat atc     1488
Leu Glu Gln Leu Lys Arg Gln Gln Ser Trp Leu Lys Asn Lys His Ile
                485                 490                 495

gac tta cgt gtc tgc ggt gtt gcc aac tcg aag gct ctg ctc acc aat     1536
Asp Leu Arg Val Cys Gly Val Ala Asn Ser Lys Ala Leu Leu Thr Asn
            500                 505                 510

gta cat ggc ctt aat ctg gaa aac tgg cag gaa gaa ctg gcg caa gcc     1584
Val His Gly Leu Asn Leu Glu Asn Trp Gln Glu Glu Leu Ala Gln Ala
        515                 520                 525

aaa gag ccg ttt aat ctc ggg cgc tta att cgc ctc gtg aaa gaa tat     1632
Lys Glu Pro Phe Asn Leu Gly Arg Leu Ile Arg Leu Val Lys Glu Tyr
    530                 535                 540

cat ctg ctg aac ccg gtc att gtt gac tgc act tcc agc cag gca gtg     1680
His Leu Leu Asn Pro Val Ile Val Asp Cys Thr Ser Ser Gln Ala Val
545                 550                 555                 560

gcg gat caa tat gcc gac ttc ctg cgc gaa ggt ttc cac gtt gtc acg     1728
Ala Asp Gln Tyr Ala Asp Phe Leu Arg Glu Gly Phe His Val Val Thr
                565                 570                 575

ccg aac aaa aag gcc aac acc tcg tcg atg gat tac tac cat cag ttg     1776
Pro Asn Lys Lys Ala Asn Thr Ser Ser Met Asp Tyr Tyr His Gln Leu
            580                 585                 590

cgt tat gcg gcg gaa aaa tcg cgg cgt aaa ttc ctc tat gac acc aac     1824

Arg Tyr Ala Ala Glu Lys Ser Arg Arg Lys Phe Leu Tyr Asp Thr Asn
        595                 600                 605

gtt ggg gct gga tta ccg gtt att gag aac ctg caa aat ctg ctc aat      1872
Val Gly Ala Gly Leu Pro Val Ile Glu Asn Leu Gln Asn Leu Leu Asn
    610                 615                 620

gca ggt gat gaa ttg atg aag ttc tcc ggc att ctt tct ggt tcg ctt      1920
Ala Gly Asp Glu Leu Met Lys Phe Ser Gly Ile Leu Ser Gly Ser Leu
625                 630                 635                 640

tct tat atc ttc ggc aag tta gac gaa ggc atg agt ttc tcc gag gcg      1968
Ser Tyr Ile Phe Gly Lys Leu Asp Glu Gly Met Ser Phe Ser Glu Ala
                645                 650                 655

acc acg ctg gcg cgg gaa atg ggt tat acc gaa ccg gac ccg cga gat      2016
Thr Thr Leu Ala Arg Glu Met Gly Tyr Thr Glu Pro Asp Pro Arg Asp
            660                 665                 670

gat ctt tct ggt atg gat gtg gcg cgt aaa cta ttg att ctc gct cgt      2064
Asp Leu Ser Gly Met Asp Val Ala Arg Lys Leu Leu Ile Leu Ala Arg
        675                 680                 685

gaa acg gga cgt gaa ctg gag ctg gcg gat att gaa att gaa cct gtg      2112
Glu Thr Gly Arg Glu Leu Glu Leu Ala Asp Ile Glu Ile Glu Pro Val
    690                 695                 700

ctg ccc gca gag ttt aac gcc gag ggt gat gtt gcc gct ttt atg gcg      2160
Leu Pro Ala Glu Phe Asn Ala Glu Gly Asp Val Ala Ala Phe Met Ala
705                 710                 715                 720

aat ctg tca caa ctc gac gat ctc ttt gcc gcg cgc gtg gcg aag gcc      2208
Asn Leu Ser Gln Leu Asp Asp Leu Phe Ala Ala Arg Val Ala Lys Ala
                725                 730                 735

cgt gat gaa gga aaa gtt ttg cgc tat gtt ggc aat att gat gaa gat      2256
Arg Asp Glu Gly Lys Val Leu Arg Tyr Val Gly Asn Ile Asp Glu Asp
            740                 745                 750

ggc gtc tgc cgc gtg aag att gcc gaa gtg gat ggt aat gat ccg ctg      2304
Gly Val Cys Arg Val Lys Ile Ala Glu Val Asp Gly Asn Asp Pro Leu
        755                 760                 765

ttc aaa gtg aaa aat ggc gaa aac gcc ctg gcc ttc tat agc cac tat      2352
Phe Lys Val Lys Asn Gly Glu Asn Ala Leu Ala Phe Tyr Ser His Tyr
    770                 775                 780

tat cag ccg ctg ccg ttg gta ctg cgc gga tat ggt gcg ggc aat gac      2400
Tyr Gln Pro Leu Pro Leu Val Leu Arg Gly Tyr Gly Ala Gly Asn Asp
785                 790                 795                 800

gtt aca gct gcc ggt gtc ttt gct gat ctg cta cgt acc ctc tca tgg      2448
Val Thr Ala Ala Gly Val Phe Ala Asp Leu Leu Arg Thr Leu Ser Trp
                805                 810                 815

aag tta gga gtc tga                                                  2463
Lys Leu Gly Val
            820


<210>  8
<211>  820
<212>  PRT
<213>  Escherichia coli

<400>  8

Met Arg Val Leu Lys Phe Gly Gly Thr Ser Val Ala Asn Ala Glu Arg
1               5                   10                  15

Phe Leu Arg Val Ala Asp Ile Leu Glu Ser Asn Ala Arg Gln Gly Gln
            20                  25                  30

Val Ala Thr Val Leu Ser Ala Pro Ala Lys Ile Thr Asn His Leu Val
        35                  40                  45

Ala Met Ile Glu Lys Thr Ile Ser Gly Gln Asp Ala Leu Pro Asn Ile
    50                  55                  60

Ser Asp Ala Glu Arg Ile Phe Ala Glu Leu Leu Thr Gly Leu Ala Ala
65                  70                  75                  80

Ala Gln Pro Gly Phe Pro Leu Ala Gln Leu Lys Thr Phe Val Asp Gln
                85                  90                  95

Glu Phe Ala Gln Ile Lys His Val Leu His Gly Ile Ser Leu Leu Gly
            100                 105                 110

Gln Cys Pro Asp Ser Ile Asn Ala Ala Leu Ile Cys Arg Gly Glu Lys
        115                 120                 125

Met Ser Ile Ala Ile Met Ala Gly Val Leu Glu Ala Arg Gly His Asn
    130                 135                 140

Val Thr Val Ile Asp Pro Val Glu Lys Leu Leu Ala Val Gly His Tyr
145                 150                 155                 160

Leu Glu Ser Thr Val Asp Ile Ala Glu Ser Thr Arg Arg Ile Ala Ala
                165                 170                 175

Ser Arg Ile Pro Ala Asp His Met Val Leu Met Ala Gly Phe Thr Ala
            180                 185                 190

Gly Asn Glu Lys Gly Glu Leu Val Val Leu Gly Arg Asn Gly Ser Asp
        195                 200                 205

Tyr Ser Ala Ala Val Leu Ala Ala Cys Leu Arg Ala Asp Cys Cys Glu
    210                 215                 220

Ile Trp Thr Asp Val Asp Gly Val Tyr Thr Cys Asp Pro Arg Gln Val
225                 230                 235                 240

Pro Asp Ala Arg Leu Leu Lys Ser Met Ser Tyr Gln Glu Ala Met Glu
                245                 250                 255

Leu Ser Tyr Phe Gly Ala Lys Val Leu His Pro Arg Thr Ile Thr Pro
            260                 265                 270

Ile Ala Gln Phe Gln Ile Pro Cys Leu Ile Lys Asn Thr Gly Asn Pro
        275                 280                 285

Gln Ala Pro Gly Thr Leu Ile Gly Ala Ser Arg Asp Glu Asp Glu Leu
    290                 295                 300

Pro Val Lys Gly Ile Ser Asn Leu Asn Asn Met Ala Met Phe Ser Val
305                 310                 315                 320

Ser Gly Pro Gly Met Lys Gly Met Val Gly Met Ala Ala Arg Val Phe
                325                 330                 335

Ala Ala Met Ser Arg Ala Arg Ile Ser Val Val Leu Ile Thr Gln Ser
            340                 345                 350

Ser Ser Glu Tyr Ser Ile Ser Phe Cys Val Pro Gln Ser Asp Cys Val
        355                 360                 365

Arg Ala Glu Arg Ala Met Gln Glu Glu Phe Tyr Leu Glu Leu Lys Glu
    370                 375                 380

Gly Leu Leu Glu Pro Leu Ala Val Thr Glu Arg Leu Ala Ile Ile Ser
385                 390                 395                 400

Val Val Gly Asp Gly Met Arg Thr Leu Arg Gly Ile Ser Ala Lys Phe
                405                 410                 415

Phe Ala Ala Leu Ala Arg Ala Asn Ile Asn Ile Val Ala Ile Ala Gln
            420                 425                 430

Gly Ser Ser Glu Arg Ser Ile Ser Val Val Val Asn Asn Asp Asp Ala
        435                 440                 445

Thr Thr Gly Val Arg Val Thr His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Leu Glu Gln Leu Lys Arg Gln Gln Ser Trp Leu Lys Asn Lys His Ile
                485                 490                 495

Asp Leu Arg Val Cys Gly Val Ala Asn Ser Lys Ala Leu Leu Thr Asn
            500                 505                 510

Val His Gly Leu Asn Leu Glu Asn Trp Gln Glu Glu Leu Ala Gln Ala
        515                 520                 525

Lys Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Ala Asp Phe Leu Arg Glu Gly Phe His Val Val Thr
                565                 570                 575

Pro Asn Lys Lys Ala Asn Thr Ser Ser Met Asp Tyr Tyr His Gln Leu
            580                 585                 590

Arg Tyr Ala Ala Glu Lys Ser Arg Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Met Lys Phe Ser Gly Ile Leu Ser Gly Ser Leu
625                 630                 635                 640

Ser Tyr Ile Phe Gly Lys Leu Asp Glu Gly Met Ser Phe Ser Glu Ala
                645                 650                 655

Thr Thr Leu Ala Arg Glu Met Gly Tyr Thr Glu Pro Asp Pro Arg Asp
            660                 665                 670

Asp Leu Ser Gly Met Asp Val Ala Arg Lys Leu Leu Ile Leu Ala Arg
        675                 680                 685

Glu Thr Gly Arg Glu Leu Glu Leu Ala Asp Ile Glu Ile Glu Pro Val
    690                 695                 700

Leu Pro Ala Glu Phe Asn Ala Glu Gly Asp Val Ala Ala Phe Met Ala
705                 710                 715                 720

Asn Leu Ser Gln Leu Asp Asp Leu Phe Ala Ala Arg Val Ala Lys Ala
                725                 730                 735

Arg Asp Glu Gly Lys Val Leu Arg Tyr Val Gly Asn Ile Asp Glu Asp
            740                 745                 750

Gly Val Cys Arg Val Lys Ile Ala Glu Val Asp Gly Asn Asp Pro Leu
        755                 760                 765

Phe Lys Val Lys Asn Gly Glu Asn Ala Leu Ala Phe Tyr Ser His Tyr
    770                 775                 780

Tyr Gln Pro Leu Pro Leu Val Leu Arg Gly Tyr Gly Ala Gly Asn Asp
785                 790                 795                 800

Val Thr Ala Ala Gly Val Phe Ala Asp Leu Leu Arg Thr Leu Ser Trp
                805                 810                 815

Lys Leu Gly Val
            820

<210>  9

<211>  2460
<212>  DNA
<213>  Serratia marcescens

<220>
<221>  CDS
<222>  (1)..(2460)
<223>  thrA-Kodierregion

<400>  9
atg cga gtg ctg aaa ttt ggc gga acc tcg gta gcg aat gcg gaa cgt        48
Met Arg Val Leu Lys Phe Gly Gly Thr Ser Val Ala Asn Ala Glu Arg
1               5                   10                  15

ttt ctg cgc gtc gcc gac atc atg gag agt aac gcg cgt cag gga cag        96
Phe Leu Arg Val Ala Asp Ile Met Glu Ser Asn Ala Arg Gln Gly Gln
            20                  25                  30

gta gcc acg gtc ttg tcc gcc ccc gca aag atc acc aac cat ctg gtg       144
Val Ala Thr Val Leu Ser Ala Pro Ala Lys Ile Thr Asn His Leu Val
        35                  40                  45

gcg atg atc gac aaa acg gtg gcg ggc cag gac att ctg ccg aat atg       192
Ala Met Ile Asp Lys Thr Val Ala Gly Gln Asp Ile Leu Pro Asn Met
    50                  55                  60

agc gac gcc gag cgg atc ttt gcc gac ctg ctg agc gga ctg gcg cag       240
Ser Asp Ala Glu Arg Ile Phe Ala Asp Leu Leu Ser Gly Leu Ala Gln
65                  70                  75                  80

gcg ctg ccg ggc ttt gaa tac gat cgt ttg aaa ggc gtg gtc gat cag       288
Ala Leu Pro Gly Phe Glu Tyr Asp Arg Leu Lys Gly Val Val Asp Gln
                85                  90                  95

gaa ttc gcg cag ctc aaa cag gtg ctg cat ggc gtc tcg ctg ctg ggg       336
Glu Phe Ala Gln Leu Lys Gln Val Leu His Gly Val Ser Leu Leu Gly
            100                 105                 110

cag tgc ccg gac agc gtg aac gcg gcg atc atc tgc cgt ggc gaa aag       384
Gln Cys Pro Asp Ser Val Asn Ala Ala Ile Ile Cys Arg Gly Glu Lys
        115                 120                 125

ctc tcc atc gcc atc atg gaa ggg gtg ttc cgc gcc aag ggt tat ccg       432
Leu Ser Ile Ala Ile Met Glu Gly Val Phe Arg Ala Lys Gly Tyr Pro
    130                 135                 140

gtc acg gtg atc aac ccg gtg gag aaa ctg ctg gcg cag ggc cac tac       480
Val Thr Val Ile Asn Pro Val Glu Lys Leu Leu Ala Gln Gly His Tyr
145                 150                 155                 160

ctg gag tcc acc gtg gac atc gcc gag tct acg ctg cgc atc gcc gcc       528
Leu Glu Ser Thr Val Asp Ile Ala Glu Ser Thr Leu Arg Ile Ala Ala
                165                 170                 175

gcg gcg atc ccg gcc gat cac atc gtg ctg atg gcc ggt ttc acc gcc       576
Ala Ala Ile Pro Ala Asp His Ile Val Leu Met Ala Gly Phe Thr Ala
            180                 185                 190

ggt aat gat aag ggc gag ctg gtg gtg ctg ggc cgc aac ggc tcc gac       624
Gly Asn Asp Lys Gly Glu Leu Val Val Leu Gly Arg Asn Gly Ser Asp
        195                 200                 205

tat tcc gcc gcg gtg ctg gcc gcc tgc ctg cgc gcc gac tgt tgc gag       672
Tyr Ser Ala Ala Val Leu Ala Ala Cys Leu Arg Ala Asp Cys Cys Glu
    210                 215                 220

atc tgg acc gac gtc gac ggc gtt tat acc tgc gat ccg cgc acc gtg       720
Ile Trp Thr Asp Val Asp Gly Val Tyr Thr Cys Asp Pro Arg Thr Val

225                 230                 235                 240

ccg gac gcc agg tta ctg aaa tcg atg tcg tac cag gaa gcg atg gag      768
Pro Asp Ala Arg Leu Leu Lys Ser Met Ser Tyr Gln Glu Ala Met Glu
                245                 250                 255

ctt tcc tat ttc ggc gcc aaa gtg tta cac ccc cgc acc atc act ccg      816
Leu Ser Tyr Phe Gly Ala Lys Val Leu His Pro Arg Thr Ile Thr Pro
            260                 265                 270

att gcc cag ttt caa atc cct tgc ctg att aaa aac acc tcc aac ccg      864
Ile Ala Gln Phe Gln Ile Pro Cys Leu Ile Lys Asn Thr Ser Asn Pro
        275                 280                 285

cag gcc ccc ggc acg ctg atc ggc aaa gac agc acc gat gcc gat atg      912
Gln Ala Pro Gly Thr Leu Ile Gly Lys Asp Ser Thr Asp Ala Asp Met
    290                 295                 300

ccg gtg aag ggc atc acc aac ctg aac aac atg gcg atg atc aac gtc      960
Pro Val Lys Gly Ile Thr Asn Leu Asn Asn Met Ala Met Ile Asn Val
305                 310                 315                 320

tcc ggc ccg ggc atg aaa ggc atg gtc ggc atg gcc gca cgg gtc ttc     1008
Ser Gly Pro Gly Met Lys Gly Met Val Gly Met Ala Ala Arg Val Phe
                325                 330                 335

gcc gtg atg tcg cgc gcc ggc att tcg gtg gtg ctg atc acc caa tcc     1056
Ala Val Met Ser Arg Ala Gly Ile Ser Val Val Leu Ile Thr Gln Ser
            340                 345                 350

tct tcc gaa tac agc atc agc ttc tgc gtg ccg cag ggt gaa ctg cag     1104
Ser Ser Glu Tyr Ser Ile Ser Phe Cys Val Pro Gln Gly Glu Leu Gln
        355                 360                 365

cgc gcc cgt cgc gcg ctg gaa gag gag ttc tat ctg gag ctg aaa gac     1152
Arg Ala Arg Arg Ala Leu Glu Glu Glu Phe Tyr Leu Glu Leu Lys Asp
    370                 375                 380

ggc gtg ctg gat ccg ctg gac gtg atg gaa cgc ctg gcg atc atc tcg     1200
Gly Val Leu Asp Pro Leu Asp Val Met Glu Arg Leu Ala Ile Ile Ser
385                 390                 395                 400

gtg gtt ggc gac ggc atg cgc acc ctg cgc ggc att tcc gca cgc ttc     1248
Val Val Gly Asp Gly Met Arg Thr Leu Arg Gly Ile Ser Ala Arg Phe
                405                 410                 415

ttc tcc gcg ctg gcg cgc gcc aac atc aat atc gtt gcc atc gcc caa     1296
Phe Ser Ala Leu Ala Arg Ala Asn Ile Asn Ile Val Ala Ile Ala Gln
            420                 425                 430

ggg tct tcc gaa cgt tcc att tcg gtg gtg gtc agc aat gat tcc gcc     1344
Gly Ser Ser Glu Arg Ser Ile Ser Val Val Val Ser Asn Asp Ser Ala
        435                 440                 445

acc acc ggc gtg cgc gtc agc cac cag atg ctg ttc aac acc gat cag     1392
Thr Thr Gly Val Arg Val Ser His Gln Met Leu Phe Asn Thr Asp Gln
    450                 455                 460

gtg atc gaa gtg ttc gtc atc ggc gtc ggc ggc gtc ggc ggg gcg ctg     1440
Val Ile Glu Val Phe Val Ile Gly Val Gly Gly Val Gly Gly Ala Leu
465                 470                 475                 480

atc gag cag atc tat cgc cag cag ccg tgg ctg aag caa aaa cat atc     1488
Ile Glu Gln Ile Tyr Arg Gln Gln Pro Trp Leu Lys Gln Lys His Ile
                485                 490                 495

gat ctg cgg gtg tgc ggc atc gcc aac tcg cgc gtg atg ctg acc aat     1536
Asp Leu Arg Val Cys Gly Ile Ala Asn Ser Arg Val Met Leu Thr Asn
            500                 505                 510

gtg cac ggc atc gcg ctg gac agc tgg cgc gac gcg ctg gcc ggc gcg     1584
Val His Gly Ile Ala Leu Asp Ser Trp Arg Asp Ala Leu Ala Gly Ala
        515                 520                 525

cag gag ccg ttc aat ctc ggc cgt ctg atc cgg ctg gtg aag gaa tat     1632
Gln Glu Pro Phe Asn Leu Gly Arg Leu Ile Arg Leu Val Lys Glu Tyr
    530                 535                 540

cac ctg ctg aac ccg gtg atc gtc gac tgt acc tcc agt cag gcg gtg     1680
His Leu Leu Asn Pro Val Ile Val Asp Cys Thr Ser Ser Gln Ala Val
545                 550                 555                 560

gcc gat cag tac gtg gac ttc ctg gcg gac ggc ttc cat gtg gtg acg     1728
Ala Asp Gln Tyr Val Asp Phe Leu Ala Asp Gly Phe His Val Val Thr
                565                 570                 575

ccg aac aaa aag gcc aac acc tcg tcg atg aac tat tat cag caa ctg     1776
Pro Asn Lys Lys Ala Asn Thr Ser Ser Met Asn Tyr Tyr Gln Gln Leu
            580                 585                 590

cgc gcg gcc gcc gcc ggt tcg cat cgc aag ttc ctg tac gac acc aac     1824
Arg Ala Ala Ala Ala Gly Ser His Arg Lys Phe Leu Tyr Asp Thr Asn
        595                 600                 605

gtc ggt gcc ggt ctg ccg gtg att gag aac ctg caa aac ctg ctg aac     1872
Val Gly Ala Gly Leu Pro Val Ile Glu Asn Leu Gln Asn Leu Leu Asn
    610                 615                 620

gcc ggt gat gaa ctg gta cgt ttc tcc ggt atc ctg tcc ggc tcg ctg     1920
Ala Gly Asp Glu Leu Val Arg Phe Ser Gly Ile Leu Ser Gly Ser Leu
625                 630                 635                 640

tcc ttt atc ttc ggc aag ctg gac gaa ggg ctg tcg ctg tcg gcg gcg     1968
Ser Phe Ile Phe Gly Lys Leu Asp Glu Gly Leu Ser Leu Ser Ala Ala
                645                 650                 655

acc ctg cag gcc cgg gcc aac ggc tac acc gag ccg gat ccg cgc gac     2016
Thr Leu Gln Ala Arg Ala Asn Gly Tyr Thr Glu Pro Asp Pro Arg Asp
            660                 665                 670

gat ctg tcc ggg atg gat gtg gcg cgt aag ctg ctg atc ctg gcg cgc     2064
Asp Leu Ser Gly Met Asp Val Ala Arg Lys Leu Leu Ile Leu Ala Arg
        675                 680                 685

gag gcc ggt tac aaa ctg gaa ctg agc gat atc gag gtc gag ccg gtg     2112
Glu Ala Gly Tyr Lys Leu Glu Leu Ser Asp Ile Glu Val Glu Pro Val
    690                 695                 700

ctg ccg ccg tcc ttc gac gcg tcg ggc gac gtg gac acc ttc ctg gcg     2160
Leu Pro Pro Ser Phe Asp Ala Ser Gly Asp Val Asp Thr Phe Leu Ala
705                 710                 715                 720

cgg ttg ccg gag ctg gat aaa gag ttc gcg cgc aac gtg gct aac gcc     2208
Arg Leu Pro Glu Leu Asp Lys Glu Phe Ala Arg Asn Val Ala Asn Ala
                725                 730                 735

gcc gag caa ggc aag gtg ctg cgc tat gtc ggc ctg atc gac gaa ggg     2256
Ala Glu Gln Gly Lys Val Leu Arg Tyr Val Gly Leu Ile Asp Glu Gly
            740                 745                 750

cgc tgc aag gtg cgc att gag gcg gtg gac ggc aac gat ccg ttg tat     2304
Arg Cys Lys Val Arg Ile Glu Ala Val Asp Gly Asn Asp Pro Leu Tyr
        755                 760                 765

aaa gtg aag aac ggc gag aac gcg ctg gcc ttc tac agc cgc tac tat     2352
Lys Val Lys Asn Gly Glu Asn Ala Leu Ala Phe Tyr Ser Arg Tyr Tyr
    770                 775                 780

cag ccg ttg ccg ctg gtg ctg cgc ggc tac ggc gcc ggt aac gat gtg     2400
Gln Pro Leu Pro Leu Val Leu Arg Gly Tyr Gly Ala Gly Asn Asp Val
785                 790                 795                 800

act gca gcg ggc gta ttc gcc gat ctg ctg cgc aca ctg tca tgg aag     2448
Thr Ala Ala Gly Val Phe Ala Asp Leu Leu Arg Thr Leu Ser Trp Lys
                805                 810                 815

ttg gga gtt taa                                                     2460
Leu Gly Val

<210>  10
<211>  819
<212>  PRT
<213>  Serratia marcescens

<400>  10

Met Arg Val Leu Lys Phe Gly Gly Thr Ser Val Ala Asn Ala Glu Arg
1               5                   10                  15

Phe Leu Arg Val Ala Asp Ile Met Glu Ser Asn Ala Arg Gln Gly Gln
            20                  25                  30

Val Ala Thr Val Leu Ser Ala Pro Ala Lys Ile Thr Asn His Leu Val
        35                  40                  45

Ala Met Ile Asp Lys Thr Val Ala Gly Gln Asp Ile Leu Pro Asn Met
    50                  55                  60

Ser Asp Ala Glu Arg Ile Phe Ala Asp Leu Leu Ser Gly Leu Ala Gln
65                  70                  75                  80

Ala Leu Pro Gly Phe Glu Tyr Asp Arg Leu Lys Gly Val Val Asp Gln
                85                  90                  95

Glu Phe Ala Gln Leu Lys Gln Val Leu His Gly Val Ser Leu Leu Gly
            100                 105                 110

Gln Cys Pro Asp Ser Val Asn Ala Ala Ile Ile Cys Arg Gly Glu Lys
        115                 120                 125

Leu Ser Ile Ala Ile Met Glu Gly Val Phe Arg Ala Lys Gly Tyr Pro
    130                 135                 140

Val Thr Val Ile Asn Pro Val Glu Lys Leu Leu Ala Gln Gly His Tyr
145                 150                 155                 160

Leu Glu Ser Thr Val Asp Ile Ala Glu Ser Thr Leu Arg Ile Ala Ala
                165                 170                 175

Ala Ala Ile Pro Ala Asp His Ile Val Leu Met Ala Gly Phe Thr Ala
            180                 185                 190

Gly Asn Asp Lys Gly Glu Leu Val Val Leu Gly Arg Asn Gly Ser Asp

195                 200                 205

Tyr Ser Ala Ala Val Leu Ala Ala Cys Leu Arg Ala Asp Cys Cys Glu
    210                 215                 220

Ile Trp Thr Asp Val Asp Gly Val Tyr Thr Cys Asp Pro Arg Thr Val
225                 230                 235                 240

Pro Asp Ala Arg Leu Leu Lys Ser Met Ser Tyr Gln Glu Ala Met Glu
                245                 250                 255

Leu Ser Tyr Phe Gly Ala Lys Val Leu His Pro Arg Thr Ile Thr Pro
            260                 265                 270

Ile Ala Gln Phe Gln Ile Pro Cys Leu Ile Lys Asn Thr Ser Asn Pro
        275                 280                 285

Gln Ala Pro Gly Thr Leu Ile Gly Lys Asp Ser Thr Asp Ala Asp Met
    290                 295                 300

Pro Val Lys Gly Ile Thr Asn Leu Asn Asn Met Ala Met Ile Asn Val
305                 310                 315                 320

Ser Gly Pro Gly Met Lys Gly Met Val Gly Met Ala Ala Arg Val Phe
                325                 330                 335

Ala Val Met Ser Arg Ala Gly Ile Ser Val Val Leu Ile Thr Gln Ser
            340                 345                 350

Ser Ser Glu Tyr Ser Ile Ser Phe Cys Val Pro Gln Gly Glu Leu Gln
        355                 360                 365

Arg Ala Arg Arg Ala Leu Glu Glu Glu Phe Tyr Leu Glu Leu Lys Asp
    370                 375                 380

Gly Val Leu Asp Pro Leu Asp Val Met Glu Arg Leu Ala Ile Ile Ser
385                 390                 395                 400

Val Val Gly Asp Gly Met Arg Thr Leu Arg Gly Ile Ser Ala Arg Phe
                405                 410                 415

Phe Ser Ala Leu Ala Arg Ala Asn Ile Asn Ile Val Ala Ile Ala Gln
            420                 425                 430

Gly Ser Ser Glu Arg Ser Ile Ser Val Val Val Ser Asn Asp Ser Ala
        435                 440                 445

Thr Thr Gly Val Arg Val Ser His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Ile Glu Gln Ile Tyr Arg Gln Gln Pro Trp Leu Lys Gln Lys His Ile
                485                 490                 495

Asp Leu Arg Val Cys Gly Ile Ala Asn Ser Arg Val Met Leu Thr Asn
            500                 505                 510

Val His Gly Ile Ala Leu Asp Ser Trp Arg Asp Ala Leu Ala Gly Ala
        515                 520                 525

Gln Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Val Asp Phe Leu Ala Asp Gly Phe His Val Val Thr
                565                 570                 575

Pro Asn Lys Lys Ala Asn Thr Ser Ser Met Asn Tyr Tyr Gln Gln Leu
            580                 585                 590

Arg Ala Ala Ala Ala Gly Ser His Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Val Arg Phe Ser Gly Ile Leu Ser Gly Ser Leu
625                 630                 635                 640

Ser Phe Ile Phe Gly Lys Leu Asp Glu Gly Leu Ser Leu Ser Ala Ala
                645                 650                 655

Thr Leu Gln Ala Arg Ala Asn Gly Tyr Thr Glu Pro Asp Pro Arg Asp
            660                 665                 670

Asp Leu Ser Gly Met Asp Val Ala Arg Lys Leu Leu Ile Leu Ala Arg
        675                 680                 685

Glu Ala Gly Tyr Lys Leu Glu Leu Ser Asp Ile Glu Val Glu Pro Val
    690                 695                 700

Leu Pro Pro Ser Phe Asp Ala Ser Gly Asp Val Asp Thr Phe Leu Ala
705                 710                 715                 720

Arg Leu Pro Glu Leu Asp Lys Glu Phe Ala Arg Asn Val Ala Asn Ala
                725                 730                 735

Ala Glu Gln Gly Lys Val Leu Arg Tyr Val Gly Leu Ile Asp Glu Gly
            740                 745                 750

Arg Cys Lys Val Arg Ile Glu Ala Val Asp Gly Asn Asp Pro Leu Tyr
        755                 760                 765

Lys Val Lys Asn Gly Glu Asn Ala Leu Ala Phe Tyr Ser Arg Tyr Tyr
    770                 775                 780

Gln Pro Leu Pro Leu Val Leu Arg Gly Tyr Gly Ala Gly Asn Asp Val
785                 790                 795                 800

Thr Ala Ala Gly Val Phe Ala Asp Leu Leu Arg Thr Leu Ser Trp Lys
                805                 810                 815

Leu Gly Val

<210>  11
<211>  1650
<212>  DNA
<213>  Escherichia coli

<220>
<221>  CDS
<222>  (1)..(1650)
<223>  yjcG-Kodierregion

<400>  11
atg aaa aga gtt ctg acg gcg ctt gcc gcc aca ctc cct ttc gca gct       48
Met Lys Arg Val Leu Thr Ala Leu Ala Ala Thr Leu Pro Phe Ala Ala
1               5                   10                  15

aac gcc gcg gat gct att agc ggg gcc gta gag cgc cag cca acg aac       96
Asn Ala Ala Asp Ala Ile Ser Gly Ala Val Glu Arg Gln Pro Thr Asn
            20                  25                  30

tgg cag gcg att att atg ttc ctg att ttc gtc gtg ttt acg ctc ggc      144
Trp Gln Ala Ile Ile Met Phe Leu Ile Phe Val Val Phe Thr Leu Gly
        35                  40                  45

att acc tac tgg gca tca aaa cgc gta cgt tct cgt agc gac tac tac      192
Ile Thr Tyr Trp Ala Ser Lys Arg Val Arg Ser Arg Ser Asp Tyr Tyr
    50                  55                  60

acc gca ggc ggc aat atc act ggc ttc cag aac ggg ctg gcg att gcc      240
Thr Ala Gly Gly Asn Ile Thr Gly Phe Gln Asn Gly Leu Ala Ile Ala
65                  70                  75                  80

ggg gac tat atg tcc gcc gcc tca ttc ttg ggg atc tcc gcg ctg gtg      288
Gly Asp Tyr Met Ser Ala Ala Ser Phe Leu Gly Ile Ser Ala Leu Val
                85                  90                  95

ttt acc tcc ggc tat gac ggc tta att tac tcg ctg ggc ttc ctg gtg      336
Phe Thr Ser Gly Tyr Asp Gly Leu Ile Tyr Ser Leu Gly Phe Leu Val
            100                 105                 110

ggc tgg ccg atc att ttg ttc ctg att gcc gaa cgt ctg cgt aac ctg      384
Gly Trp Pro Ile Ile Leu Phe Leu Ile Ala Glu Arg Leu Arg Asn Leu
        115                 120                 125

ggg cgc tac acc ttt gcc gat gtg gcc tct tac cgt ctg aaa caa ggg      432
Gly Arg Tyr Thr Phe Ala Asp Val Ala Ser Tyr Arg Leu Lys Gln Gly
    130                 135                 140

ccg att cgt att ctt tcg gcc tgt ggt tct ctg gtg gtg gtg gcg ctt 480
Pro Ile Arg Ile Leu Ser Ala Cys Gly Ser Leu Val Val Val Ala Leu
145                 150                 155                 160

tac ctt atc gcc cag atg gtg ggc gca ggt aaa ctg atc gag ctg ctg 528
Tyr Leu Ile Ala Gln Met Val Gly Ala Gly Lys Leu Ile Glu Leu Leu
                165                 170                 175

ttt ggc ctt aac tat cac att gcg gtg gtg ctg gtc ggc gtg ctg atg 576
Phe Gly Leu Asn Tyr His Ile Ala Val Val Leu Val Gly Val Leu Met
            180                 185                 190

atg atg tac gtc ctg ttc ggc ggc atg ctg gcg acc acc tgg gtg caa 624
Met Met Tyr Val Leu Phe Gly Gly Met Leu Ala Thr Thr Trp Val Gln
        195                 200                 205

att atc aaa gcc gtg ctg ttg ctg ttc ggt gcc agc ttt atg gcc ttt 672
Ile Ile Lys Ala Val Leu Leu Leu Phe Gly Ala Ser Phe Met Ala Phe
    210                 215                 220

atg gtg atg aaa cac gtc ggc ttt agc ttc aac aat ctg ttc agt gaa 720
Met Val Met Lys His Val Gly Phe Ser Phe Asn Asn Leu Phe Ser Glu
225                 230                 235                 240

gcg atg gcg gta cac ccg aaa ggt gtc gac atc atg aag ccg ggc ggg 768
Ala Met Ala Val His Pro Lys Gly Val Asp Ile Met Lys Pro Gly Gly
                245                 250                 255

ctg gtg aaa gat ccg atc tcc gcg ctc tct ctg ggt ctg gga ctg atg 816
Leu Val Lys Asp Pro Ile Ser Ala Leu Ser Leu Gly Leu Gly Leu Met
            260                 265                 270

ttt ggt acg gcg ggc ttg ccg cac att ctg atg cgc ttc ttt aca gtc 864
Phe Gly Thr Ala Gly Leu Pro His Ile Leu Met Arg Phe Phe Thr Val
        275                 280                 285

agc gat gcc cgc gaa gca cgt aag agc gtg ttc tac gcc acc ggg ttt 912
Ser Asp Ala Arg Glu Ala Arg Lys Ser Val Phe Tyr Ala Thr Gly Phe
    290                 295                 300

atg ggc tac ttc tat att ctg acc ttt att atc ggc ttc ggc gcg atc 960
Met Gly Tyr Phe Tyr Ile Leu Thr Phe Ile Ile Gly Phe Gly Ala Ile
305                 310                 315                 320

atg ctg gtt ggt gcg aat ccg gaa tat aaa gac gcg gcg ggc cat ctg 1008
Met Leu Val Gly Ala Asn Pro Glu Tyr Lys Asp Ala Ala Gly His Leu
                325                 330                 335

att ggt ggt aac aac atg gcg gcc gtt cac ctg gcg aat gca gtg ggc 1056
Ile Gly Gly Asn Asn Met Ala Ala Val His Leu Ala Asn Ala Val Gly
            340                 345                 350

ggc aac ctg ttc ctc ggt ttt att tca gcg gtt gct ttc gcc act atc 1104
Gly Asn Leu Phe Leu Gly Phe Ile Ser Ala Val Ala Phe Ala Thr Ile
        355                 360                 365

ctc gcg gtg gtt gcg ggt ctg acg ctg gcg ggc gca tcc gcg gtt tcg 1152
Leu Ala Val Val Ala Gly Leu Thr Leu Ala Gly Ala Ser Ala Val Ser
    370                 375                 380

cat gac ttg tac gct aac gtc ttc aaa aaa ggc gcg acc gaa cgt gaa 1200
His Asp Leu Tyr Ala Asn Val Phe Lys Lys Gly Ala Thr Glu Arg Glu
385                 390                 395                 400

gag ctg cgg gta tca aaa atc acc gta ctg atc ctc ggc gtg att gcg 1248
Glu Leu Arg Val Ser Lys Ile Thr Val Leu Ile Leu Gly Val Ile Ala
                405                 410                 415

att atc ctc ggc gtg ctg ttt gag aat cag aac atc gcc ttt atg gtg 1296

Ile Ile Leu Gly Val Leu Phe Glu Asn Gln Asn Ile Ala Phe Met Val
            420                 425                 430

ggg ctg gcg ttt gcc atc gcg gcg agc tgt aac ttc ccg atc att ctg      1344
Gly Leu Ala Phe Ala Ile Ala Ala Ser Cys Asn Phe Pro Ile Ile Leu
        435                 440                 445

ctt tct atg tac tgg tcg aaa ctg acc acg cgt ggc gcg atg atg ggt      1392
Leu Ser Met Tyr Trp Ser Lys Leu Thr Thr Arg Gly Ala Met Met Gly
    450                 455                 460

ggc tgg ctg ggg ctg att acc gca gta gta ctg atg atc ctc ggc ccg      1440
Gly Trp Leu Gly Leu Ile Thr Ala Val Val Leu Met Ile Leu Gly Pro
465                 470                 475                 480

acg att tgg gta cag atc ctt ggt cac gaa aaa gcc atc ttc ccg tat      1488
Thr Ile Trp Val Gln Ile Leu Gly His Glu Lys Ala Ile Phe Pro Tyr
                485                 490                 495

gaa tac ccg gcg ctg ttc tct atc acc gtg gca ttc ctc ggc atc tgg      1536
Glu Tyr Pro Ala Leu Phe Ser Ile Thr Val Ala Phe Leu Gly Ile Trp
            500                 505                 510

ttc ttc tcg gca acc gat aac tca gcg gaa ggc gcg cgt gag cgt gaa      1584
Phe Phe Ser Ala Thr Asp Asn Ser Ala Glu Gly Ala Arg Glu Arg Glu
        515                 520                 525

ctg ttc cgc gcg cag ttt atc cgc tcc cag acc ggc ttt ggc gtt gag      1632
Leu Phe Arg Ala Gln Phe Ile Arg Ser Gln Thr Gly Phe Gly Val Glu
    530                 535                 540

caa ggc cgc gcg cat taa                                              1650
Gln Gly Arg Ala His
545


<210>  12
<211>  549
<212>  PRT
<213>  Escherichia coli

<400>  12

Met Lys Arg Val Leu Thr Ala Leu Ala Ala Thr Leu Pro Phe Ala Ala
1               5                   10                  15


Asn Ala Ala Asp Ala Ile Ser Gly Ala Val Glu Arg Gln Pro Thr Asn
            20                  25                  30


Trp Gln Ala Ile Ile Met Phe Leu Ile Phe Val Val Phe Thr Leu Gly
        35                  40                  45


Ile Thr Tyr Trp Ala Ser Lys Arg Val Arg Ser Arg Ser Asp Tyr Tyr
    50                  55                  60


Thr Ala Gly Gly Asn Ile Thr Gly Phe Gln Asn Gly Leu Ala Ile Ala
65                  70                  75                  80


Gly Asp Tyr Met Ser Ala Ala Ser Phe Leu Gly Ile Ser Ala Leu Val
                85                  90                  95


Phe Thr Ser Gly Tyr Asp Gly Leu Ile Tyr Ser Leu Gly Phe Leu Val
            100                 105                 110

Gly Trp Pro Ile Ile Leu Phe Leu Ile Ala Glu Arg Leu Arg Asn Leu
        115                 120                 125

Gly Arg Tyr Thr Phe Ala Asp Val Ala Ser Tyr Arg Leu Lys Gln Gly
    130                 135                 140

Pro Ile Arg Ile Leu Ser Ala Cys Gly Ser Leu Val Val Val Ala Leu
145                 150                 155                 160

Tyr Leu Ile Ala Gln Met Val Gly Ala Gly Lys Leu Ile Glu Leu Leu
                165                 170                 175

Phe Gly Leu Asn Tyr His Ile Ala Val Val Leu Val Gly Val Leu Met
            180                 185                 190

Met Met Tyr Val Leu Phe Gly Gly Met Leu Ala Thr Thr Trp Val Gln
        195                 200                 205

Ile Ile Lys Ala Val Leu Leu Leu Phe Gly Ala Ser Phe Met Ala Phe
    210                 215                 220

Met Val Met Lys His Val Gly Phe Ser Phe Asn Asn Leu Phe Ser Glu
225                 230                 235                 240

Ala Met Ala Val His Pro Lys Gly Val Asp Ile Met Lys Pro Gly Gly
                245                 250                 255

Leu Val Lys Asp Pro Ile Ser Ala Leu Ser Leu Gly Leu Gly Leu Met
            260                 265                 270

Phe Gly Thr Ala Gly Leu Pro His Ile Leu Met Arg Phe Phe Thr Val
        275                 280                 285

Ser Asp Ala Arg Glu Ala Arg Lys Ser Val Phe Tyr Ala Thr Gly Phe
    290                 295                 300

Met Gly Tyr Phe Tyr Ile Leu Thr Phe Ile Ile Gly Phe Gly Ala Ile
305                 310                 315                 320

Met Leu Val Gly Ala Asn Pro Glu Tyr Lys Asp Ala Ala Gly His Leu
                325                 330                 335

Ile Gly Gly Asn Asn Met Ala Ala Val His Leu Ala Asn Ala Val Gly
            340                 345                 350

Gly Asn Leu Phe Leu Gly Phe Ile Ser Ala Val Ala Phe Ala Thr Ile
        355                 360                 365

Leu Ala Val Val Ala Gly Leu Thr Leu Ala Gly Ala Ser Ala Val Ser
    370                 375                 380

His Asp Leu Tyr Ala Asn Val Phe Lys Lys Gly Ala Thr Glu Arg Glu
385                 390                 395                 400

Glu Leu Arg Val Ser Lys Ile Thr Val Leu Ile Leu Gly Val Ile Ala
                405                 410                 415

Ile Ile Leu Gly Val Leu Phe Glu Asn Gln Asn Ile Ala Phe Met Val
            420                 425                 430

Gly Leu Ala Phe Ala Ile Ala Ala Ser Cys Asn Phe Pro Ile Ile Leu
        435                 440                 445

Leu Ser Met Tyr Trp Ser Lys Leu Thr Thr Arg Gly Ala Met Met Gly
    450                 455                 460

Gly Trp Leu Gly Leu Ile Thr Ala Val Val Leu Met Ile Leu Gly Pro
465                 470                 475                 480

Thr Ile Trp Val Gln Ile Leu Gly His Glu Lys Ala Ile Phe Pro Tyr
                485                 490                 495

Glu Tyr Pro Ala Leu Phe Ser Ile Thr Val Ala Phe Leu Gly Ile Trp
            500                 505                 510

Phe Phe Ser Ala Thr Asp Asn Ser Ala Glu Gly Ala Arg Glu Arg Glu
        515                 520                 525

Leu Phe Arg Ala Gln Phe Ile Arg Ser Gln Thr Gly Phe Gly Val Glu
    530                 535                 540

Gln Gly Arg Ala His
545

<210>  13
<211>  1035
<212>  DNA
<213>  Escherichia coli

<220>
<221>  CDS
<222>  (1)..(1035)
<223>  yibD-Kodierregion

<400>  13
atg atg aac agc acc aat aaa ctt agt gtt att att ccg tta tat aat       48
Met Met Asn Ser Thr Asn Lys Leu Ser Val Ile Ile Pro Leu Tyr Asn
1               5                   10                  15

gcg ggc gat gat ttc cgc act tgt atg gaa tct tta att acg caa acc       96
Ala Gly Asp Asp Phe Arg Thr Cys Met Glu Ser Leu Ile Thr Gln Thr
            20                  25                  30

tgg act gct ctg gaa atc att att att aac gat ggt tca acg gat aat      144
Trp Thr Ala Leu Glu Ile Ile Ile Ile Asn Asp Gly Ser Thr Asp Asn
        35                  40                  45

tct gtt gaa ata gca aag tat tac gca gaa aac tat ccg cac gtt cgt      192
Ser Val Glu Ile Ala Lys Tyr Tyr Ala Glu Asn Tyr Pro His Val Arg
    50                  55                  60

ttg ttg cat cag gcg aat gct ggc gca tcg gtg gcg cgt aat cgt ggg      240
Leu Leu His Gln Ala Asn Ala Gly Ala Ser Val Ala Arg Asn Arg Gly
65                  70                  75                  80

att gaa gtg gca acg ggc aaa tat gtc gct ttt gtc gat gct gac gat      288
Ile Glu Val Ala Thr Gly Lys Tyr Val Ala Phe Val Asp Ala Asp Asp
                85                  90                  95

gaa gtc tat ccc acc atg tac gaa acg ctg atg acc atg gcg tta gag      336
Glu Val Tyr Pro Thr Met Tyr Glu Thr Leu Met Thr Met Ala Leu Glu
            100                 105                 110

gac gac ctc gac gtg gcg cag tgc aac gct gac tgg tgt ttt cgt gaa      384
Asp Asp Leu Asp Val Ala Gln Cys Asn Ala Asp Trp Cys Phe Arg Glu
        115                 120                 125

acg gga gaa acc tgg caa tcc atc ccc acc gat cgc ctt cgc tca acc      432
Thr Gly Glu Thr Trp Gln Ser Ile Pro Thr Asp Arg Leu Arg Ser Thr
    130                 135                 140

ggc gta tta acc ggc ccg gac tgg ctg cgg atg ggg ctt tct tcg cgc      480
Gly Val Leu Thr Gly Pro Asp Trp Leu Arg Met Gly Leu Ser Ser Arg
145                 150                 155                 160

cgt tgg act cac gtt gtc tgg atg ggg gtt tat cgc cgt gat gtt att      528
Arg Trp Thr His Val Val Trp Met Gly Val Tyr Arg Arg Asp Val Ile
                165                 170                 175

gtt aaa aat aac att aaa ttt att gcc gga tta cat cat cag gat att      576
Val Lys Asn Asn Ile Lys Phe Ile Ala Gly Leu His His Gln Asp Ile
            180                 185                 190

gtc tgg aca aca gaa ttc atg ttt aac gcg ctg cgt gcg cga tat acc      624
Val Trp Thr Thr Glu Phe Met Phe Asn Ala Leu Arg Ala Arg Tyr Thr
        195                 200                 205

gag caa tca tta tat aaa tat tat ctg cat aat acg tca gtg agt cgg      672
Glu Gln Ser Leu Tyr Lys Tyr Tyr Leu His Asn Thr Ser Val Ser Arg
    210                 215                 220

ttg cat aga caa ggg aat aaa aac ctt aat tat caa cgt cac tat att      720
Leu His Arg Gln Gly Asn Lys Asn Leu Asn Tyr Gln Arg His Tyr Ile
225                 230                 235                 240

aag att acc cgc ctg ctg gag aaa tta aat cga aat tat gcc gac aaa      768
Lys Ile Thr Arg Leu Leu Glu Lys Leu Asn Arg Asn Tyr Ala Asp Lys
                245                 250                 255

att atg att tat ccg gaa ttt cat cag caa ata act tac gag gca ttg      816
Ile Met Ile Tyr Pro Glu Phe His Gln Gln Ile Thr Tyr Glu Ala Leu
            260                 265                 270

cgt gtt tgc cat gcg gtg cgc aaa gag ccg gat att ctt acc cgc caa      864
Arg Val Cys His Ala Val Arg Lys Glu Pro Asp Ile Leu Thr Arg Gln
        275                 280                 285

cgg atg att gcc gag ata ttt act tcc ggt atg tat aag cgc ctg att      912
Arg Met Ile Ala Glu Ile Phe Thr Ser Gly Met Tyr Lys Arg Leu Ile
    290                 295                 300

acc aat gtg cgc agc gtg aag gtc ggt tac cag gcg tta ctg tgg tct      960
Thr Asn Val Arg Ser Val Lys Val Gly Tyr Gln Ala Leu Leu Trp Ser
305                 310                 315                 320

ttc cgc tta tgg caa tgg cgc gac aaa acg cgg tcg cac cat cgc att     1008

Phe Arg Leu Trp Gln Trp Arg Asp Lys Thr Arg Ser His His Arg Ile
                325                 330                 335

acg cgt agc gcc ttt aat ttg cgc tag                                 1035
Thr Arg Ser Ala Phe Asn Leu Arg
            340

<210>  14
<211>  344
<212>  PRT
<213>  Escherichia coli

<400>  14

Met Met Asn Ser Thr Asn Lys Leu Ser Val Ile Ile Pro Leu Tyr Asn
1               5                   10                  15

Ala Gly Asp Asp Phe Arg Thr Cys Met Glu Ser Leu Ile Thr Gln Thr
            20                  25                  30

Trp Thr Ala Leu Glu Ile Ile Ile Ile Asn Asp Gly Ser Thr Asp Asn
        35                  40                  45

Ser Val Glu Ile Ala Lys Tyr Tyr Ala Glu Asn Tyr Pro His Val Arg
    50                  55                  60

Leu Leu His Gln Ala Asn Ala Gly Ala Ser Val Ala Arg Asn Arg Gly
65                  70                  75                  80

Ile Glu Val Ala Thr Gly Lys Tyr Val Ala Phe Val Asp Ala Asp Asp
                85                  90                  95

Glu Val Tyr Pro Thr Met Tyr Glu Thr Leu Met Thr Met Ala Leu Glu
            100                 105                 110

Asp Asp Leu Asp Val Ala Gln Cys Asn Ala Asp Trp Cys Phe Arg Glu
        115                 120                 125

Thr Gly Glu Thr Trp Gln Ser Ile Pro Thr Asp Arg Leu Arg Ser Thr
    130                 135                 140

Gly Val Leu Thr Gly Pro Asp Trp Leu Arg Met Gly Leu Ser Ser Arg
145                 150                 155                 160

Arg Trp Thr His Val Val Trp Met Gly Val Tyr Arg Arg Asp Val Ile
                165                 170                 175

Val Lys Asn Asn Ile Lys Phe Ile Ala Gly Leu His His Gln Asp Ile
            180                 185                 190

Val Trp Thr Thr Glu Phe Met Phe Asn Ala Leu Arg Ala Arg Tyr Thr
        195                 200                 205

Glu Gln Ser Leu Tyr Lys Tyr Tyr Leu His Asn Thr Ser Val Ser Arg
    210                 215                 220

Leu His Arg Gln Gly Asn Lys Asn Leu Asn Tyr Gln Arg His Tyr Ile
225                 230                 235                 240

Lys Ile Thr Arg Leu Leu Glu Lys Leu Asn Arg Asn Tyr Ala Asp Lys
                245                 250                 255

Ile Met Ile Tyr Pro Glu Phe His Gln Gln Ile Thr Tyr Glu Ala Leu
            260                 265                 270

Arg Val Cys His Ala Val Arg Lys Glu Pro Asp Ile Leu Thr Arg Gln
        275                 280                 285

Arg Met Ile Ala Glu Ile Phe Thr Ser Gly Met Tyr Lys Arg Leu Ile
    290                 295                 300

Thr Asn Val Arg Ser Val Lys Val Gly Tyr Gln Ala Leu Leu Trp Ser
305                 310                 315                 320

Phe Arg Leu Trp Gln Trp Arg Asp Lys Thr Arg Ser His His Arg Ile
                325                 330                 335

Thr Arg Ser Ala Phe Asn Leu Arg
            340

<210>  15
<211>  1332
<212>  DNA
<213>  Escherichia coli

<220>
<221>  CDS
<222>  (1)..(1332)
<223>  yaaU-Kodierregion

<400>  15
atg caa ccg tcc aga aac ttt gac gat ctc aaa ttc tcc tct att cac        48
Met Gln Pro Ser Arg Asn Phe Asp Asp Leu Lys Phe Ser Ser Ile His
1               5                   10                  15

cgc cgc att ttg ctg tgg gga agc ggt ggt ccg ttt ctg gat ggt tat        96
Arg Arg Ile Leu Leu Trp Gly Ser Gly Gly Pro Phe Leu Asp Gly Tyr
            20                  25                  30

gta ctg gta atg att ggc gtg gcg ctg gag caa ctg acg ccg gcg ctg       144
Val Leu Val Met Ile Gly Val Ala Leu Glu Gln Leu Thr Pro Ala Leu
        35                  40                  45

aaa ctg gac gct gac tgg att ggc ttg ctg ggc gcg gga acg ctc gcc       192
Lys Leu Asp Ala Asp Trp Ile Gly Leu Leu Gly Ala Gly Thr Leu Ala
    50                  55                  60

ggg ctg ttc gtt ggc aca tcg ctg ttt ggt tat att tcc gat aaa gtc       240
Gly Leu Phe Val Gly Thr Ser Leu Phe Gly Tyr Ile Ser Asp Lys Val
65                  70                  75                  80

gga cgg cgc aaa atg ttc ctc att gat atc atc gcc atc ggc gtg ata       288
Gly Arg Arg Lys Met Phe Leu Ile Asp Ile Ile Ala Ile Gly Val Ile
                85                  90                  95

tcg gtg gcg acg atg ttt gtt tca tcc ccc gtc gaa ctg ttg gtg atg 336
Ser Val Ala Thr Met Phe Val Ser Ser Pro Val Glu Leu Leu Val Met
            100                 105                 110

cgg gta ctt atc ggc att gtc atc ggt gca gat tat ccc atc gcc acc 384
Arg Val Leu Ile Gly Ile Val Ile Gly Ala Asp Tyr Pro Ile Ala Thr
        115                 120                 125

tca atg atc acc gag ttc tcc agt acc cgt cag cgg gcg ttt tcc atc 432
Ser Met Ile Thr Glu Phe Ser Ser Thr Arg Gln Arg Ala Phe Ser Ile
    130                 135                 140

agc ttt att gcc gcg atg tgg tat gtc ggc gcg acc tgt gcc gat ctg 480
Ser Phe Ile Ala Ala Met Trp Tyr Val Gly Ala Thr Cys Ala Asp Leu
145                 150                 155                 160

gtc ggc tac tgg ctt tat gat gtg gaa ggc ggc tgg cgc tgg atg ctg 528
Val Gly Tyr Trp Leu Tyr Asp Val Glu Gly Gly Trp Arg Trp Met Leu
                165                 170                 175

ggt agc gcg gcg atc ccc tgt ttg ttg att ttg att ggt cga ttc gaa 576
Gly Ser Ala Ala Ile Pro Cys Leu Leu Ile Leu Ile Gly Arg Phe Glu
            180                 185                 190

ctg cct gaa tct ccc cgc tgg tta tta cgc aaa ggg cga gta aaa gag 624
Leu Pro Glu Ser Pro Arg Trp Leu Leu Arg Lys Gly Arg Val Lys Glu
        195                 200                 205

tgc gaa gag atg atg atc aaa ctg ttt ggc gaa ccg gtg gct ttc gat 672
Cys Glu Glu Met Met Ile Lys Leu Phe Gly Glu Pro Val Ala Phe Asp
    210                 215                 220

gaa gag cag ccg cag caa acc cgt ttt cgc gat ctg ttt aat cgc cgc 720
Glu Glu Gln Pro Gln Gln Thr Arg Phe Arg Asp Leu Phe Asn Arg Arg
225                 230                 235                 240

cat ttt cct ttt gtt ctg ttt gtt gcc gcc atc tgg acc tgc cag gtg 768
His Phe Pro Phe Val Leu Phe Val Ala Ala Ile Trp Thr Cys Gln Val
                245                 250                 255

atc cca atg ttc gcc att tac acc ttt ggc ccg caa atc gtt ggt ttg 816
Ile Pro Met Phe Ala Ile Tyr Thr Phe Gly Pro Gln Ile Val Gly Leu
            260                 265                 270

ttg gga ttg ggg gtt ggc aaa aac gcg gca cta ggg aat gtg gtg att 864
Leu Gly Leu Gly Val Gly Lys Asn Ala Ala Leu Gly Asn Val Val Ile
        275                 280                 285

agc ctg ttc ttt atg ctc ggc tgt att ccg ccg atg ctg tgg tta aac 912
Ser Leu Phe Phe Met Leu Gly Cys Ile Pro Pro Met Leu Trp Leu Asn
    290                 295                 300

act gcc gga cgg cgt cca ttg ttg att ggc agc ttt gcc atg atg acg 960
Thr Ala Gly Arg Arg Pro Leu Leu Ile Gly Ser Phe Ala Met Met Thr
305                 310                 315                 320

ctg gcg ctg gcg gtt ttg ggg cta atc ccg gat atg ggg atc tgg ctg 1008
Leu Ala Leu Ala Val Leu Gly Leu Ile Pro Asp Met Gly Ile Trp Leu
                325                 330                 335

gta gtg atg gcc ttt gcg gtg tat gcc ttt ttc tct ggc ggg ccg ggt 1056
Val Val Met Ala Phe Ala Val Tyr Ala Phe Phe Ser Gly Gly Pro Gly
            340                 345                 350

aat ttg cag tgg ctc tat cct aat gaa ctc ttc ccg aca gat atc cgc 1104
Asn Leu Gln Trp Leu Tyr Pro Asn Glu Leu Phe Pro Thr Asp Ile Arg
        355                 360                 365

gcc tct gcc gtg ggc gtg att atg tcc tta agt cgt att ggc acc att     1152
Ala Ser Ala Val Gly Val Ile Met Ser Leu Ser Arg Ile Gly Thr Ile
    370                 375                 380

gtt tcg acc tgg gca cta ccg atc ttt atc aat aat tac ggt atc agt     1200
Val Ser Thr Trp Ala Leu Pro Ile Phe Ile Asn Asn Tyr Gly Ile Ser
385                 390                 395                 400

aac acg atg cta atg ggg gcg ggt atc tcg ctg ttt ggc ttg ttg att     1248
Asn Thr Met Leu Met Gly Ala Gly Ile Ser Leu Phe Gly Leu Leu Ile
                405                 410                 415

tcc gta gcg ttt gcc ccg gag act cga ggg atg tca ctg gcg cag acc     1296
Ser Val Ala Phe Ala Pro Glu Thr Arg Gly Met Ser Leu Ala Gln Thr
            420                 425                 430

agc aat atg acg atc cgc ggg cag aga atg ggg taa                     1332
Ser Asn Met Thr Ile Arg Gly Gln Arg Met Gly
        435                 440

<210>  16
<211>  443
<212>  PRT
<213>  Escherichia coli

<400>  16

Met Gln Pro Ser Arg Asn Phe Asp Asp Leu Lys Phe Ser Ser Ile His
1               5                   10                  15

Arg Arg Ile Leu Leu Trp Gly Ser Gly Gly Pro Phe Leu Asp Gly Tyr
            20                  25                  30

Val Leu Val Met Ile Gly Val Ala Leu Glu Gln Leu Thr Pro Ala Leu
        35                  40                  45

Lys Leu Asp Ala Asp Trp Ile Gly Leu Leu Gly Ala Gly Thr Leu Ala
    50                  55                  60

Gly Leu Phe Val Gly Thr Ser Leu Phe Gly Tyr Ile Ser Asp Lys Val
65                  70                  75                  80

Gly Arg Arg Lys Met Phe Leu Ile Asp Ile Ile Ala Ile Gly Val Ile
                85                  90                  95

Ser Val Ala Thr Met Phe Val Ser Ser Pro Val Glu Leu Leu Val Met
            100                 105                 110

Arg Val Leu Ile Gly Ile Val Ile Gly Ala Asp Tyr Pro Ile Ala Thr
        115                 120                 125

Ser Met Ile Thr Glu Phe Ser Ser Thr Arg Gln Arg Ala Phe Ser Ile
    130                 135                 140

Ser Phe Ile Ala Ala Met Trp Tyr Val Gly Ala Thr Cys Ala Asp Leu
145                 150                 155                 160

Val Gly Tyr Trp Leu Tyr Asp Val Glu Gly Gly Trp Arg Trp Met Leu

                165                 170                 175

Gly Ser Ala Ala Ile Pro Cys Leu Leu Ile Leu Ile Gly Arg Phe Glu
            180                 185                 190

Leu Pro Glu Ser Pro Arg Trp Leu Leu Arg Lys Gly Arg Val Lys Glu
        195                 200                 205

Cys Glu Glu Met Met Ile Lys Leu Phe Gly Glu Pro Val Ala Phe Asp
    210                 215                 220

Glu Glu Gln Pro Gln Gln Thr Arg Phe Arg Asp Leu Phe Asn Arg Arg
225                 230                 235                 240

His Phe Pro Phe Val Leu Phe Val Ala Ala Ile Trp Thr Cys Gln Val
                245                 250                 255

Ile Pro Met Phe Ala Ile Tyr Thr Phe Gly Pro Gln Ile Val Gly Leu
            260                 265                 270

Leu Gly Leu Gly Val Gly Lys Asn Ala Ala Leu Gly Asn Val Val Ile
        275                 280                 285

Ser Leu Phe Phe Met Leu Gly Cys Ile Pro Pro Met Leu Trp Leu Asn
    290                 295                 300

Thr Ala Gly Arg Arg Pro Leu Leu Ile Gly Ser Phe Ala Met Met Thr
305                 310                 315                 320

Leu Ala Leu Ala Val Leu Gly Leu Ile Pro Asp Met Gly Ile Trp Leu
                325                 330                 335

Val Val Met Ala Phe Ala Val Tyr Ala Phe Phe Ser Gly Gly Pro Gly
            340                 345                 350

Asn Leu Gln Trp Leu Tyr Pro Asn Glu Leu Phe Pro Thr Asp Ile Arg
        355                 360                 365

Ala Ser Ala Val Gly Val Ile Met Ser Leu Ser Arg Ile Gly Thr Ile
    370                 375                 380

Val Ser Thr Trp Ala Leu Pro Ile Phe Ile Asn Asn Tyr Gly Ile Ser
385                 390                 395                 400

Asn Thr Met Leu Met Gly Ala Gly Ile Ser Leu Phe Gly Leu Leu Ile
                405                 410                 415

Ser Val Ala Phe Ala Pro Glu Thr Arg Gly Met Ser Leu Ala Gln Thr
            420                 425                 430

Ser Asn Met Thr Ile Arg Gly Gln Arg Met Gly
        435                 440

<210>  17
<211>  882
<212>  DNA
<213>  Escherichia coli


<220>
<221>  CDS
<222>  (1)..(882)
<223>  yddG-Kodierregion

<400>  17
atg aca cga caa aaa gca acg ctc ata ggg ctg ata gcg atc gtc ctg        48
Met Thr Arg Gln Lys Ala Thr Leu Ile Gly Leu Ile Ala Ile Val Leu
1               5                   10                  15

tgg agc acg atg gta gga ttg att cgc ggt gtc agt gag ggg ctc ggc        96
Trp Ser Thr Met Val Gly Leu Ile Arg Gly Val Ser Glu Gly Leu Gly
            20                  25                  30

ccg gtc ggc ggc gca gct gct atc tat tca tta agc ggg ctg ctg tta       144
Pro Val Gly Gly Ala Ala Ala Ile Tyr Ser Leu Ser Gly Leu Leu Leu
        35                  40                  45

atc ttc acg gtt gga ttt ccg cgt att cgg caa atc ccg aaa ggc tat       192
Ile Phe Thr Val Gly Phe Pro Arg Ile Arg Gln Ile Pro Lys Gly Tyr
    50                  55                  60

tta ctc gcc ggg agt ctg tta ttc gtc agc tat gaa atc tgt ctg gcg       240
Leu Leu Ala Gly Ser Leu Leu Phe Val Ser Tyr Glu Ile Cys Leu Ala
65                  70                  75                  80

ctt tcc tta ggg tat gcg gcg acc cat cat cag gcg att gaa gtg ggt       288
Leu Ser Leu Gly Tyr Ala Ala Thr His His Gln Ala Ile Glu Val Gly
                85                  90                  95

atg gtg aac tat ctg tgg ccc agc ctg aca att ctc ttt gcc att ctg       336
Met Val Asn Tyr Leu Trp Pro Ser Leu Thr Ile Leu Phe Ala Ile Leu
            100                 105                 110

ttt aat ggt cag aaa acc aac tgg ttg att gta cct gga tta tta tta       384
Phe Asn Gly Gln Lys Thr Asn Trp Leu Ile Val Pro Gly Leu Leu Leu
        115                 120                 125

gcc ctc gtc ggc gtc tgt tgg gtg tta ggc ggt gac aat ggg tta cat       432
Ala Leu Val Gly Val Cys Trp Val Leu Gly Gly Asp Asn Gly Leu His
    130                 135                 140

tat gat gaa atc atc aat aat atc acc acc agc cca ttg agt tat ttc       480
Tyr Asp Glu Ile Ile Asn Asn Ile Thr Thr Ser Pro Leu Ser Tyr Phe
145                 150                 155                 160

ctg gcg ttc att ggt gcg ttt atc tgg gca gcc tat tgc aca gta acg       528
Leu Ala Phe Ile Gly Ala Phe Ile Trp Ala Ala Tyr Cys Thr Val Thr
                165                 170                 175

aat aaa tac gca cgc gga ttt aat gga att acc gtt ttt gtc ctg cta       576
Asn Lys Tyr Ala Arg Gly Phe Asn Gly Ile Thr Val Phe Val Leu Leu
            180                 185                 190

acg gga gca agt ctg tgg gtt tac tat ttt ctt acg cca caa cca gaa       624
Thr Gly Ala Ser Leu Trp Val Tyr Tyr Phe Leu Thr Pro Gln Pro Glu
        195                 200                 205

atg ata ttt agc acg ccc gtc atg att aaa ctc atc tct gcg gca ttt       672
Met Ile Phe Ser Thr Pro Val Met Ile Lys Leu Ile Ser Ala Ala Phe
    210                 215                 220

acc tta gga ttt gct tat gct gca tgg aat gtc ggt ata ttg cat ggc      720
Thr Leu Gly Phe Ala Tyr Ala Ala Trp Asn Val Gly Ile Leu His Gly
225                 230                 235                 240

aat gtc acc att atg gcg gta ggt tcg tat ttt acg cct gta ctt tcc      768
Asn Val Thr Ile Met Ala Val Gly Ser Tyr Phe Thr Pro Val Leu Ser
                245                 250                 255

tca gcg ctt gca gcc gtg ctg ctc agc gcc ccg ctg tcg ttc tcg ttc      816
Ser Ala Leu Ala Ala Val Leu Leu Ser Ala Pro Leu Ser Phe Ser Phe
            260                 265                 270

tgg caa ggc gcg ctg atg gtc tgc ggc ggt tcc ctg ctc tgc tgg ctg      864
Trp Gln Gly Ala Leu Met Val Cys Gly Gly Ser Leu Leu Cys Trp Leu
        275                 280                 285

gcg aca cgt cgt ggt taa                                              882
Ala Thr Arg Arg Gly
    290


<210>  18
<211>  293
<212>  PRT
<213>  Escherichia coli

<400>  18

Met Thr Arg Gln Lys Ala Thr Leu Ile Gly Leu Ile Ala Ile Val Leu
1               5                   10                  15


Trp Ser Thr Met Val Gly Leu Ile Arg Gly Val Ser Glu Gly Leu Gly
            20                  25                  30


Pro Val Gly Gly Ala Ala Ala Ile Tyr Ser Leu Ser Gly Leu Leu Leu
        35                  40                  45


Ile Phe Thr Val Gly Phe Pro Arg Ile Arg Gln Ile Pro Lys Gly Tyr
    50                  55                  60


Leu Leu Ala Gly Ser Leu Leu Phe Val Ser Tyr Glu Ile Cys Leu Ala
65                  70                  75                  80


Leu Ser Leu Gly Tyr Ala Ala Thr His His Gln Ala Ile Glu Val Gly
                85                  90                  95


Met Val Asn Tyr Leu Trp Pro Ser Leu Thr Ile Leu Phe Ala Ile Leu
            100                 105                 110


Phe Asn Gly Gln Lys Thr Asn Trp Leu Ile Val Pro Gly Leu Leu Leu
        115                 120                 125


Ala Leu Val Gly Val Cys Trp Val Leu Gly Gly Asp Asn Gly Leu His
    130                 135                 140


Tyr Asp Glu Ile Ile Asn Asn Ile Thr Thr Ser Pro Leu Ser Tyr Phe
145                 150                 155                 160

Leu Ala Phe Ile Gly Ala Phe Ile Trp Ala Ala Tyr Cys Thr Val Thr
                165                 170                 175

Asn Lys Tyr Ala Arg Gly Phe Asn Gly Ile Thr Val Phe Val Leu Leu
            180                 185                 190

Thr Gly Ala Ser Leu Trp Val Tyr Tyr Phe Leu Thr Pro Gln Pro Glu
        195                 200                 205

Met Ile Phe Ser Thr Pro Val Met Ile Lys Leu Ile Ser Ala Ala Phe
    210                 215                 220

Thr Leu Gly Phe Ala Tyr Ala Ala Trp Asn Val Gly Ile Leu His Gly
225                 230                 235                 240

Asn Val Thr Ile Met Ala Val Gly Ser Tyr Phe Thr Pro Val Leu Ser
                245                 250                 255

Ser Ala Leu Ala Ala Val Leu Leu Ser Ala Pro Leu Ser Phe Ser Phe
            260                 265                 270

Trp Gln Gly Ala Leu Met Val Cys Gly Gly Ser Leu Leu Cys Trp Leu
        275                 280                 285

Ala Thr Arg Arg Gly
    290